# EUROPEAN PATENT APPLICATION

(11) **EP 2 458 005 A1**
(43) Date of publication of application: **30.05.2012**
(21) Application number: 10802326.8
(22) Date of filing: 22.07.2010
(51) Int. Cl.: C12N 15/113, A61K 31/7088, A61P 1/16, A61P 3/04, A61P 3/06, A61P 3/10, A61P 9/00, A61P 9/10, A61P 9/12, A61P 11/00, A61P 19/06, A61P 35/00, A61P 43/00, C12N 15/09

(54) **FGF21 CIS-ELEMENT BINDING SUBSTANCE**

(30) Priority: 23.07.2009 JP 2009172584
(71) Applicant: Galaxy Pharma Inc., Akita 010-0951 (JP)
(72) Inventor: ADACHI, Shungo, Tokyo 105-0023 (JP); NISHI, Kazunori, Kanagawa 251-0012 (JP); UMEMOTO, Tadashi, Kanagawa 251-0012 (JP); NOGAMI, Masahiro, Kanagawa 251-0012 (JP); NAKAO, Shoichi, Kanagawa 251-0012 (JP)
(74) Representative: Duckworth, Timothy John
(86) International application number: PCT/JP2010/062383
(87) International publication number: WO 2011/010706

(57) **Abstract**

The present invention provides a substance capable of binding to at least a portion of a cis-element of the mRNA of FGF21 to inhibit the binding of a cis-element binding factor to the cis-element, particularly an oligonucleotide containing a base sequence complementary to at least a portion of an AU-rich element, as well as a medicament containing the substance, particularly the oligonucleotide, particularly an agent for the prophylaxis or treatment of lifestyle-related diseases such as antiobesity agent and the like.

SEQUENCE LISTING

<110>  Takeda Pharmaceutical Company Limited
       Galaxy Pharma Inc.

<120>  FGF21 cis-element binding substance

<130>  091578

<150>  JP 2009-172584
<151>  2009-07-23

<160>  38

<170>  PatentIn version 3.5

<210>  1
<211>  940
<212>  DNA
<213>  Homo sapiens

<400>  1
ctgtcagctg aggatccagc cgaaagagga gccaggcact caggccacct gagtctactc      60

acctggacaa ctggaatctg gcaccaattc taaaccactc agcttctccg agctcacacc     120

ccggagatca cctgaggacc cgagccattg atggactcgg acgagaccgg gttcgagcac     180

tcaggactgt gggtttctgt gctggctggt cttctgctgg gagcctgcca ggcacacccc     240

atccctgact ccagtcctct cctgcaattc gggggccaag tccggcagcg gtacctctac     300

acagatgatg cccagcagac agaagcccac ctggagatca gggaggatgg gacggtgggg     360

ggcgctgctg accagagccc cgaaagtctc ctgcagctga aagccttgaa gccgggagtt     420

attcaaatct tgggagtcaa gacatccagg ttcctgtgcc agcggccaga tggggccctg     480

tatggatcgc tccactttga ccctgaggcc tgcagcttcc gggagctgct tcttgaggac     540

ggatacaatg tttaccagtc cgaagcccac ggcctcccgc tgcacctgcc agggaacaag     600

tccccacacc gggaccctgc accccgagga ccagctcgct tcctgccact accaggcctg     660

ccccccgcac tcccggagcc acccggaatc ctggcccccc agccccccga tgtgggctcc     720

tcggaccctc tgagcatggt gggaccttcc cagggccgaa gccccagcta cgcttcctga     780

agccagaggc tgtttactat gacatctcct ctttatttat taggttattt atcttattta     840

tttttttatt tttcttactt gagataataa agagttccag aggagaaaaa aaaaaaaaaa     900

aaaaaaaaaa aaaaaaaaaa aaaaaaaaaa aaaaaaaaaa                           940


<210>  2
<211>  12
<212>  DNA
<213>  Artificial Sequence

<220>
<223>  Antisense oligonucleotide

<400>  2
taacctaata aa                                                          12

<210> 3
<211> 12
<212> DNA
<213> Artificial Sequence

<220>
<223> Antisense oligonucleotide

<400> 3
aaataagata aa 12


<210> 4
<211> 11
<212> DNA
<213> Artificial Sequence

<220>
<223> Antisense oligonucleotide

<400> 4
agatgaataa a 11


<210> 5
<211> 3022
<212> DNA
<213> Homo sapiens

<400> 5
cggagtcaga aaggcgaggg gcgccgggaa ctggcgtgtg ggactccaga caggagaggc 60

tgcgccttcc ccgcaccggg accttcgcga cacaccagat cctcgcccct ggctcgcgcg 120

aacgcacagg atgaccacca ccctcgtgtc tgccaccatc ttcgacttga gcgaagtttt 180

atgcaagggt aacaagatgc tcaactatag tgctcccagt gcagggggtt gcctgctgga 240

cagaaaggca gtgggcaccc ctgctggtgg gggcttccct cggaggcact cagtcaccct 300

gcccagctcc aagttccacc agaaccagct cctcagcagc ctcaagggtg agccagcccc 360

cgctctgagc tcgcgagaca gccgcttccg agaccgctcc ttctcggaag ggggcgagcg 420

gctgctgccc acccagaagc agcccggggg cggccaggtc aactccagcc gctacaagac 480

ggagctgtgc cgcccctttg aggaaaacgg tgcctgtaag tacggggaca agtgccagtt 540

cgcacacggc atccacgagc tccgcagcct gacccgccac cccaagtaca agacggagct 600

gtgccgcacc ttccacacca tcggcttttg cccctacggg ccccgctgcc acttcatcca 660

caacgctgaa gagcgccgtg ccctggccgg ggcccgggac ctctccgctg accgtccccg 720

cctccagcat agctttagct ttgctgggtt tcccagtgcc gctgccaccg ccgctgccac 780

cgggctgctg gacagcccca cgtccatcac cccaccccct attctgagcg ccgatgacct 840

cctgggctca cctaccctgc ccgatggcac caataaccct tttgccttct ccagccagga 900

gctggcaagc ctctttgccc ctagcatggg gctgcccggg ggtggctccc cgaccacctt 960

cctcttccgg cccatgtccg agtcccctca catgtttgac tctcccccca gccctcagga 1020

ttctctctcg gaccaggagg gctacctgag cagctccagc agcagccaca gtggctcaga 1080

ctccccgacc ttggacaact caagacgcct gcccatcttc agcagacttt ccatctcaga 1140

tgactaagcc agggtaggga gggacctcct gcctactcca gcccctaccc tgcacccaca 1200

tcccataccc tcttctccct acccatccca ttccccacag gccctacatt aacaaggtta 1260

agctcaaccc ctttccccca gcacctcaga atgtgccctc cctctccccc tcataacccc 1320

acctaacata aggacaagtc aatttgtcag tagcttcttc tggcttgaaa ccccctccct 1380

ggattttata gcccacttac catgcataac agacaagtcc catattttgt cagtagatgc 1440

cttttttttt ccggcttaag ccttaagtgc caaatcacaa gagaaaaagc agtaacagtt 1500

tacagaagca acttagtgcc ttgtaatcta actttgtcac tgtgactaca ttacctcttc 1560

agcgccagag ggcacccgtg ggcctcccgg agcctctgcc catggcgggg tggagacccg 1620

gaaccagcag ccccctccac tggcgacaca actgcacctt ccctcatttc agtctcccgc 1680

acacttattc ctcctcccct cttcccggtg gcacctctcc acctgtaccc gcccccccac 1740

cccccccacc cctgcccctt ggaagagttg ttgccagacc agggttttgg gggaaacctg 1800

tcttgacatt caaaaccttt ttcttcccga tctgaacccc tgttgactaa tcttgcctgg 1860

gtttgtgtag gtctgcagga aggaaggctg aaaaagcgga cgaagatttt gacttaagtg 1920

ggactttgtg atttaatttt ttcttttttt taagtgggga ggaaggggaa gctagatgga 1980

ctaggagaga cttgattttg gtgctaaagt tccccagttc atatgtgaca tctttttaaa 2040

aaaaataaca acaaaaaaaa aatgagagaa aagctaaaaa aaaaaaagta aggggtgagc 2100

agttaatggt attcattcca catacaatat ctgtgtaaaa cgatttcctg tagaagtagc 2160

tttaatggtt tttgctctag aataccgtag gtctatcctt agagcactca cgccatgctt 2220

tcttccctgg gttttaaact tcatataact ttcagaaatt ggagagcaaa aattttgctt 2280

gtcactgcac atcaatataa aaaagcttat ttaacttatc aaaacgtatt tattgccaaa 2340

ctatgctttt ttttgttaat tttgttcata tttatcggga tgacaaatcc atagaatata 2400

ttcttttatg ttaaattatg atcttcatat taatcttaaa attttgtgac gtgtcttttt 2460

cctttttttc cacagtttta atatattatt cttcaacgac attttttgta actttacact 2520

tttttggtta ttttatttta aaaaaatgaa aaattaattt aaaaaaatgc aaaaaactgt 2580

tggattattt attttagaaa ttcccccctt tgtgttggac tgcaaattga gtttctttct 2640

ctttaggcct ttcacaacta ggactgagaa tgtatgtaaa agttctgtga cagtacagaa 2700

ggaaaacaac tttttatgta tagcttctaa aaggggaaaa aaaaaaaaag agaaaccctt 2760

tgacttccac gtgcccatct caagacattc cactcacaga tttgaggttc tggattccag 2820

gtctggagtt ttccaatgtt aatgtaaaca gaactggcac acacacatta agatgaatgt 2880

aattattatt cctcttgctg gtcactaccg tcgctttcta tttctctttc tttgtgtgaa 2940

tttatttaaa agaaaaaaaa actttttgta acgactattt gcagtttaaa aatcaataaa 3000

ccccgttttt tcaagaaaca tt 3022

<210> 6
<211> 3702
<212> DNA
<213> Homo sapiens

<400> 6
gagttccagc agtccgcgag ctgccgtcgg ctccgcgggg ggggcgggcc gggcaccccg 60

gggcgcggag gagcgctcct cgcttctctc cttcccccct gccgcactcc gccggaccct 120

cccgccggcc cgcgccgctg cactcgccct ctcctctcgc cccccggcaa actttcggcc 180

cctccccgcc cctcgcccgt tattcgtcgt ggctcaagcc cggccacgcc gccccaaggg 240

ctcctcccga cctcccggcc tgccgctccg gccactgcgg gatccagaaa catgtcgacc 300

acacttctgt ccgccttcta cgatgtcgac ttcttgtgca agacagagaa atccctggcc 360

aacctcaacc tgaacaacat gctggacaag aaggcggtgg ggacgcctgt ggccgccgcc 420

cccagctcgg gcttcgcgcc gggattcctc cgacggcact cggccagcaa cctgcatgca 480

ctcgcccacc ccgcgcccag ccccggcagc tgctcgccca agttcccggg cgccgctaac 540

ggcagcagct gcggcagcgc ggcggccggc ggtccgacct cctacggcac ccttaaggag 600

ccgtcggggg gcggcggcac agccctgctc aacaaggaga acaaattccg ggaccgctcg 660

tttagcgaga acggcgatcg cagccagcac ctcctgcacc tgcagcagca gcagaagggg 720

ggcggcggct cccagatcaa ctccacgcgc tacaagaccg agctgtgccg gcccttcgag 780

gagagcggca cgtgcaagta cggcgaaaag tgccagttcg cgcatggctt ccacgagctg 840

cgcagcctga ctcgccatcc gaagtacaag accgagctgt gccgcacctt tcataccatc 900

ggcttctgcc cctatgggcc gcgctgccac ttcatccaca acgcggacga gcggcggccc 960

gcgccgtcgg ggggcgcctc cggggacctg cgtgcctttg gcacgcgcga tgcgttgcac 1020

ctgggcttcc cgcgggagcc gcggcccaag ttgcaccaca gcctcagctt ctcgggcttc 1080

ccgtcgggcc accatcagcc cccgggcggc ctcgagtcgc cgctgctgct cgacagcccc 1140

acgtcgcgca cgccgccgcc gccctcctgc tcttcggcct cgtcctgctc ctcctccgcc 1200

tcctcctgtt cctcggcctc cgcggcctcc acgccctcgg gcgccccgac atgctgcgcc 1260

tccgcggcgg ccgcggctgc ggccgctctg ctgtacggca ccgggggcgc cgaggacctg 1320

ctggcgccgg gggccccgtg cgcggcctgc tcgtcggcct cgtgcgccaa caacgccttc 1380

gccttcggtc cggagctcag cagcctcatc acgccgctcg ccatccagac ccacaacttt 1440

gccgccgtgg ccgccgccgc ctactaccgc agtcagcagc agcagcagca gcagggcctg 1500

gcgccccccg cgcagccgcc ggcgccgccc agcgcgaccc tccccgccgg ggccgccgca 1560

cctccctcgc cgcccttcag cttccagctg ccgcgccgcc tgtccgactc gcccgtgttc 1620

gacgcgcccc ccagcccccc ggactcgctg tcggaccgcg acagctacct aagcggctcc 1680

ctgagctccg gcagcctcag cggctctgag tctcccagcc tcgaccctgg ccgccgcctg 1740

ccaatcttca gccgcctctc catctccgac gactgaggca agagggcgcc agtgaggagg 1800

aagggaaggc ggttcagaga tgttggagga cacccctcgc catctcgccc ttgctggggg 1860

cacgggagtg gggggggtga catgggccct aggcagactg caagcccgac cgagcacttg 1920

gactcgaact ctgtgccggg aggggccccc acccctcctt tttcggtttc ctcttgtctt 1980

ttttttttta tttttattac gaagtttcat tctttttgag caaaaaagtc gaactttttc 2040

tgttgaacaa aatattcaca acagggcagt tgtgatacga atagaacaaa aaaaaaaaaa 2100

aaacacttaa actttgttag gactccgatg agtttgggac ttcaggaaaa atcaacccag 2160

caccagcagc taccaaccac cattccatct cttcacttga acagcattag ttaagtccag 2220

atgtgggaac ccttctcttg gaagaagttc ctaattgtgt ctcagaccgg tgtaaacaaa 2280

ccagccagcc gccaccttgc taaacctata agctttttaa aatccaatat attctgccaa 2340

gaatatgcct tgatagttag ccctcagccc ataggtgttt tttgtttttt aacagaatta 2400

tatatgtctg ggggtgaaaa aacccttgca ttccaaaggt ccatactggt tacttggttt 2460

cattgccacc acttagtgga tgttcagttt agaaccattt tgtctgctcc ctctggaagc 2520

cttgcgcaga gcttactttg taattgttgg agaataactg ctgaattttt agctgttttg 2580

agttgattcg caccactgca ccacaactca atatgaaaac tatttaactt atttattatc 2640

ttgtgaaaag tatacaatga aaattttgtt catactgtat ttatcaagta tgatgaaaag 2700

caatagatat atattctttt attatgttaa attatgattg ccattattaa tcggcaaaat 2760

gtggagtgta tgttcttttc acagtaatat atgccttttg taacttcact tggttatttt 2820

attgtaaatg agtacaaaat tcttaattta agagattgta tgtaatattt atttcattaa 2880

tttctttcct tgtttacgta aattttgaaa gattgcatga tttcttgaca gaaatcgatc 2940

ttgatgctgt ggaagtagtt tgaggaacat cctatgagtt ttcttagaat gtataaaggt 3000

tgtagcccat ccaacttcaa agaaaaaaat gaccacatac tttgcaatca ggctgaaatg 3060

tggcatgctt ttctaattcc aactttataa actagcaaaa aagtgtttgc ttattccacc 3120

agttctactg tgacatactc gagtataaag acatgtagca ataacgggga gtgggggggg 3180

agtctcacag tgcctttgga agggcccgaa cttgccttaa atcttcctca accaaataag 3240

tattttatta gtgcttgaga gaatctgaat gtaggatggg ttcaactgca caaaaggaaa 3300

agatttttac cacttttttt atatagatat aaagtgaagc aaccgcctta gtgctgaaat 3360

atgtagtaca tgaatatgcc ttgtttaatt acagaaaatt ccaaaacttg tactattttt 3420

ttttccatgt agaaaggcag gaatgtctcc taagctttcc tggacagcag atgaatgagc 3480

ggtagcttta gtttgtacgt aggtacagtt ggagcactat atgtactctc tggactactt 3540

tggacagaag taggtttttg aatgtaacaa gataagtcaa cttgagttgt aatatatttt 3600

ggggaatcag ctcactacaa attgtgactg taaacattgt actgtaaatg ttttgtagtt 3660

ttcccccaat aaaatttttg ggaaaaaaag gtaaaaaaaa aa 3702

<210> 7
<211> 2985
<212> DNA
<213> Mus musculus

<400> 7
gggagagtca gaaaggcgag gggcgccggg agcaggcgtg tgggactcct gaccggagag 60

ccggaggctg cgccttcccc gcaccgggac cttcacgaca caccagatcc tagtccttgc 120

cccgtgcgaa cgcccacgat gaccaccacc ctcgtgtccg ccaccatttt tgacttgagc 180

gaagttttat gcaagggtaa caagatgctc aactacagca ctcccagcgc tgggggctgc 240

ctgctggaca ggaaggcagt gggcacccct gctggcgggg gcttccctcg caggcactcg 300

gtcactctgc ccagctccaa gttccatcag aaccagcttc tcagcagcct taagggtgag 360

ccggccccgt ccctgagctc acgcgacagc cgctttcgag accgctcttt ctccgaaggg 420

ggcgagcggc tgctgcccac ccagaagcag cctgggagcg gccaggtcaa ctccagccgc 480

tacaagacgg agctgtgccg tcccttcgaa gaaaacggtg cctgtaagta cggggacaag 540

tgccagttcg cgcatggcat ccacgagctc cgcagcctga cccgccaccc caagtacaag 600

acggagctgt gccgcacctt ccacaccatc ggcttttgcc cgtacggccc ccgctgccac 660

ttcattcata acgccgagga gcgacgcgcc ctggcggggg gccgagacct ctccgctgac 720

cgtccccgcc tccagcatag ctttagcttt gctgggtttc ccagtgccgc tgccaccgcc 780

gctgccacgg ggctgctgga cagccccaca tccatcaccc caccccctat cctgagcgcc 840

gatgacctct tgggctcacc tactctgccc gatggcacca ataacccctt cgccttttcc 900

agccaggagc tggcgagcct ctttgctcct agcatggggc tgcctggggg aggctccccc 960

accactttcc tcttccggcc catgtccgaa tcccctcaca tgtttgactc tccccccagc 1020

cctcaggatt ctctctcgga ccacgagggc tatctgagca gctccagctc cagccacagt 1080

ggctcagact cccctacctt ggacaactca agacgcctgc ccattttcag cagactctcc 1140

atctcagatg actaagccag ggtagggagg gacccccccc atgcctcctt cacctctcca 1200

ccccatctct tcccttcacc tccccacccc ctaactttcc ctcaaacccc acattgatac 1260

atttaagctc agcccctttc ccagaacaac cttggtatgt taccctcccc ccacataagg 1320

acaagtcaat ttgttggtag cttctggctt gaaaccctct ccctccattt catagcccac 1380

ttaaccacgc ataacagagt tccatctttt gtcagtagat gccttttttt accccccccc 1440

accccccccg gcttaagccc ttaagtgcca aatcacaaaa gaaaaaaagc agtaaccgtt 1500

tacagaagca acttagtgcc ttgtaatcta actttgtcac tgtgactaca ttacctcttc 1560

aacgccaggg ggcacccgtg ggcctcccca gagcctctgc ccgtggggtt gggggtgggg 1620

gtggggtgac ccgcaaccag cagctccccc agctggccac acaagtgcac ctttctttcg 1680

gtctcccgca cactcttcct cttccttata gttactcacc tccctcagag agttgttgct 1740

ggacttgggg tttttttggg gggaggggtc tattttgact ttcaaaacct tttacttccc 1800

agcccgaacc cctgttgact aatcttgcct gggtttgtgt aggtctccag ggacaaaggt 1860

tgaacaagtt ggacgaaggt tttgacataa gtgggacttc gtgatttaat ttctttcttt 1920

cttttctttt ttttttaagt ggggaggaag gggaaactag atggactatg agagacttga 1980

ttttggtgct aaagttcccc aattcatatg tgacatctta aaaatgacaa caaatgagag 2040

aaaagctaaa aacaacaaaa aaaaacatgt aaggggtgag cagttaatgg tctgcattcc 2100

acatacaata tctgtgtaaa acgatttcct gtagaagtag ctttaatgga ttttgctcta 2160

caatacctta ggtctacctt agagccccca cacaatgctt ttttccctgg gtttaaactt 2220

catctaactt tcagaaattg gagagcaaaa atgttgctta tcactgcaca tcaatataaa 2280

aaagcttatt taacttatca aaacgtattt attgccaaac tatgcttttt ttgttaattt 2340

tgttcatatt tatcgggatg acaaatccat agaatatatt cttttatgtt aaattatgat 2400

cttcatatta atcttaaaat tttgtgacgt gtctttcctt ttttccacag ttttaatata 2460

ttattcttca acaacatttt tgtaacttta cacttttttt ggttatttta ttttaaaaaa 2520

atgaaaaatt aatttaaaaa aatgcaaaaa actgttggat tatttatttt agaaattctc 2580

ccctttgtgt tggactgcaa attgagtttc tttctcctta ggcctttcac aggtaggact 2640

gagaatgtat gtataagttc tgtgacagta cagaaggaaa accaccattt tatgtatagc 2700

ttctaaaagg gaaaacaaaa aaagagaaaa accctttgaa ttccatgtgc ccatctcaag 2760

acattccgct cgcagatttg tggttctgga ttccaggttg gagttttcca atgttgacat 2820

aaacaactgg cgcacacaca taaagatgaa tgtaattatt attcctcttg ctggtcacta 2880

ccgtcgcttt ctatttctct ttctttgtgt gaatttattt aaaagaaaaa aaaaactttt 2940

tgtaacgact atttgcagtt taaaaatcaa taaaccccgt ttttc 2985

<210> 8
<211> 3537
<212> DNA
<213> Mus musculus

<400> 8
gagttccacc agtccgcgag ctgccgacgg ctcgggcggg gggcgggccg gggcgccgag 60

ctgcgctctc gcttctcctc tgccccccac tccgcgccac ggcctcccgc cgccgcttcc 120

tcggccgcac aagcacaact ttccgtccct ccttggccct cgcccgttat tcatcttggt 180

tcgagtctcg ccgtaccgag ggcacctccc aacctccacg cttgcaattt cgaccattac 240

aggacccaga aaaatgtcga ccacacttct gtcacccttc tacgatatcg acttcttgtg 300

caagacggag aaatccctgg caaacctcaa tctgaacaac atgctggaca agaaggcggt 360

agggacgccc gtggctgctg cccccagctc gagcttcact ccgggcttcc tgcgacgcca 420

ctccgccagc aacctccacg cgcttgccca ccctgtgccc agtccgggca gctgctcgcc 480

gaagttcccg ggcgccccta acggcggcgg cagcagctgt ggccccgcag gcggaggcgg 540

tctggcctcc tacggccagc tcaaggagcc ttcggggggc agcggcacgg cgctggtcac 600

caaggagagc aaatttcggg accgctcgtt cagcgagaac ggggagcgca gccagcacct 660

cctgcacctg cagcagcagc agaagggggg cagcggctcc cagatcaact ccacgcgcta 720

caagacggag ctgtgccggc ccttcgagga gagcggcacg tgcaagtacg gcgagaagtg 780

ccagttcgcg cacggcttcc acgagctgcg cagcctcacc cggcacccca agtacaagac 840

agagctgtgc cgcaccttcc acaccatcgg cttctgcccc tacggcccgc gctgccactt 900

catccacaac gcggacgagc ggcggcccgc gccgtcgggg ggcggcggcg cctccgggga 960

cctgcgagcg tttggcgcac gcgacgcgct acacctaggc ttcgcccgcg agccgcggcc 1020

caagctgcat cacagcctca gtttctccgg cttcccgtcg ggccaccacc agccgccggg 1080

gggcctggag tcgccgctgc tgctcgacag ccccacatcg cgcacgccgc cgccgccctc 1140

ctcctcggcc tcgtcctgtt cctcctcggc ttcttcctgc tcttccgcct cggcggcctc 1200

cacgccctcg ggcgccccga cgtgctgcgc cacggcggcg gcggccgcgc tgctctacgg 1260

ccccgggggc gcggaggacc tgctgagccc cggagcgccg tgcgcctcct gctcgtcctc 1320

cggcgctaac aacgccttcg ctttcggccc ggagctgagc agcctcatca cgccgctcgc 1380

catccagacc cacaacttcg ccgccgcggc cgccgccgcc tactaccgca accagcagca 1440

gggcctgacc ggtcccgcgc cacccccggc acagcccccc gcggcccccg cgccgccctc 1500

cccgcccttc ggcttccagc tgccgcgccg tctgtccgag tcgcccgtgt tcgacgcgcc 1560

ccccagcccc ccggactcgc tctcggaccg cgacagctac ctgagcggct cgctgagctc 1620

cggcagcctc agcggctccg agtcccccag cctggaccct ggccgtcgcc tacccatctt 1680

cagccgcctc tccatctccg acgactgagg caagggggcg cccgcgaggg gttgggaaag 1740

gcacagcggg ggccaccggt tggggagggc gacactgcgg cagacttgga ggcccctccg 1800

agcacttgga ctcgaactct gccggaaggg gccccgcccc taccctttcg gttctctcgt 1860

tttttttgtt ttttattatt atttttatta ttattattat tgtaaagatt cgttcttgtt 1920

gataaaagcc aacgaacttt ttcgtatgga tgaacaaaat attcacaaca gggcggttgt 1980

gatgcggaaa gaacaaaaga taccaaacac ttaggtctca gatgagtttg aaactctagt 2040

tttagaagaa aaaaatcagc ccagcaccag cagctaccaa ccaccattcc atctcttctt 2100

tcactcgaaa gccttagaaa cagtccagat gtgagaactc ttggaagggg ttccaagacc 2160

agtgtgagcc agccggctgc cacctcccta aactataagc ttctagaact caaaataatg 2220

ccaggaacat gccttgattc gagtcgctcg ctctcagcct cctttgtggt ggttgtttac 2280

cttgtttttg ttttttagac aaagttctgt atgtctgggg gtggggaagc ccttgcattc 2340

caaagtttga taacgggctc attgccacca cgtagtgtgg gtgtttagct tagaaccatt 2400

tcctctgctc tctggaagct atgggcagag ctcagttcat agttgttggg aaataagtgc 2460

ttcattttta gttgttctga gttgatccgc accactgcac cacaactcaa tatgaaaaac 2520

tatttaactt atttattatc ttgtgaaaag tatacattga ggatgttgtc catactgtat 2580

ttatcgagta tgatgaaagc aatagatata tattctttta ttatgttaaa ttataattgc 2640

cattattaat cggcaaaatg tggagtgtgt tcttttcaca gtaatatatg ccttttgtaa 2700

cttcacttgg ttattttatt gtaaatgagt aagaaaaatc ttaatttaag agattgtatg 2760

taatatttat ttcattaatt tctttccttg tttacgtaaa tttcgaaaga tcgcatgaag 2820

aaaccatgca atcttgatgc tgtggaagtg gtttgaggaa cattttgagg ttttcttaga 2880

atgtataatg gttgtagccc atccaacttt aaagagagaa aggtgagact gcagtcaggc 2940

tcacgtgtgg cttgcttctc tacatccagc tttataaacc accagctcta ctgtgacatt 3000

gattatagag acatgtagca ataatggggg aaatggtctc agtgccttca tagggcccga 3060

acttgcctta aatctttctc aaccagataa gtatttcatt cgtgcttgag aatttgaatg 3120

tcggacgggt tcacctgcac aaaaagaaaa gattttttac cacttttttt ttactatagc 3180

tatgaagtga agaggccaca tcttagcgct gaaatgttgg tggtacatga atatgccttg 3240

ttcaatgaca gtccaaaaac ttgtactatt atttttcaat gtaaagaggc aggaatgtct 3300

ccggctttcc tggatggcag ataaatgagc agttgtttag tttgtatgta ggtacagttg 3360

gagcaccgcg tgtgcactct ggactacttt gacagaagta ggtttttgaa tgtaacaaga 3420

ttaagtcaac ttgagttgta atatattttg gggaatcagt tcactacaaa ttgtgactgt 3480

aaacattgta ctgtaaatgt tttgtagttt cccccaataa aattttttgg aaaaaaa 3537

<210> 9
<211> 947
<212> DNA
<213> Mus musculus

<400> 9
agacagcctt agtgtcttct cagctgggga ttcaacacag gagaaacagc cattcacttt 60

gcctgagccc cagtctgaac ctgacccatc cctgctgggc accggagtca gaacacaatt 120

ccagctgcct tggctcctca gccgctcgct tgccaggggc tctcccgaac ggagcgcagc 180

cctgatggaa tggatgagat ctagagttgg gaccctggga ctgtgggtcc gactgctgct 240

ggctgtcttc ctgctggggg tctaccaagc ataccccatc cctgactcca gccccctcct 300

ccagtttggg ggtcaagtcc ggcagaggta cctctacaca gatgacgacc aagacactga 360

agcccacctg gagatcaggg aggatggaac agtggtaggc gcagcacacc gcagtccaga 420

aagtctcctg gagctcaaag ccttgaagcc aggggtcatt caaatcctgg gtgtcaaagc 480

ctctaggttt ctttgccaac agccagatgg agctctctat ggatcgcctc actttgatcc     540

tgaggcctgc agcttcagag aactgctgct ggaggacggt tacaatgtgt accagtctga     600

agcccatggc ctgcccctgc gtctgcctca gaaggactcc ccaaaccagg atgcaacatc     660

ctggggacct gtgcgcttcc tgcccatgcc aggcctgctc cacgagcccc aagaccaagc     720

aggattcctg cccccagagc ccccagatgt gggctcctct gaccccctga gcatggtaga     780

gcctttacag ggccgaagcc ccagctatgc gtcctgactc ttcctgaatc tagggctgtt     840

tctttttggg tttccactta tttattacgg gtatttatct tatttattta ttttagtttt     900

tttttcttac ttggaataat aaagagtctg aaagaaaaat gtgtgtt                  947

<210>  10
<211>  22
<212>  DNA
<213>  Artificial Sequence

<220>
<223>  Primer

<400>  10
tggatcagca agcaggagta tg                                              22

<210>  11
<211>  18
<212>  DNA
<213>  Artificial Sequence

<220>
<223>  Primer

<400>  11
gcatttgcgg tggacgat                                                   18

<210>  12
<211>  21
<212>  DNA
<213>  Artificial Sequence

<220>
<223>  Primer

<400>  12
tgaagccggg agttattcaa a                                               21

<210>  13
<211>  16
<212>  DNA
<213>  Artificial Sequence

<220>
<223>  Primer

<400>  13
gccgctggca caggaa                                                     16

<210> 14
<211> 19
<212> DNA
<213> Artificial Sequence

<220>
<223> Primer

<400> 14
ccagttcgcc atggatgac 19


<210> 15
<211> 17
<212> DNA
<213> Artificial Sequence

<220>
<223> Primer

<400> 15
atgccggagc cgttgtc 17


<210> 16
<211> 25
<212> DNA
<213> Artificial Sequence

<220>
<223> Primer

<400> 16
gtacctctac acagatgacg accaa 25


<210> 17
<211> 21
<212> DNA
<213> Artificial Sequence

<220>
<223> Primer

<400> 17
cgcctaccac tgttccatcc t 21


<210> 18
<211> 18
<212> DNA
<213> Artificial Sequence

<220>
<223> Antisense oligonucleotide

<400> 18
aaataaataa gataaata 18


<210> 19
<211> 16
<212> DNA
<213> Artificial Sequence

<220>
<223>  Antisense oligonucleotide

<400>  19
ataaataaga taaata                                                        16


<210>  20
<211>  14
<212>  DNA
<213>  Artificial Sequence

<220>
<223>  Antisense oligonucleotide

<400>  20
ataaataaga taaa                                                          14


<210>  21
<211>  14
<212>  DNA
<213>  Artificial Sequence

<220>
<223>  Antisense oligonucleotide

<400>  21
aaataagata aata                                                          14


<210>  22
<211>  12
<212>  DNA
<213>  Artificial Sequence

<220>
<223>  Antisense oligonucleotide

<400>  22
aaataaataa ga                                                            12


<210>  23
<211>  12
<212>  DNA
<213>  Artificial Sequence

<220>
<223>  Antisense oligonucleotide

<400>  23
ataaataaga ta                                                            12


<210>  24
<211>  12
<212>  DNA
<213>  Artificial Sequence

<220>
<223>  Antisense oligonucleotide

<400>  24
ataagataaa ta                                                            12

<210>  25
<211>  18
<212>  DNA
<213>  Homo sapiens

<400>  25
tatttatctt atttattt                                                    18


<210>  26
<211>  70
<212>  RNA
<213>  Homo sapiens

<400>  26
uguuuacuau gacaucuccu cuuuauuuau uagguuauuu aucuuauuua uuuuuuuauu      60

uuucuuacuu                                                             70


<210>  27
<211>  18
<212>  DNA
<213>  Artificial Sequence

<220>
<223>  Primer

<400>  27
cctggcaccc agcacaat                                                    18


<210>  28
<211>  20
<212>  DNA
<213>  Artificial Sequence

<220>
<223>  Primer

<400>  28
gccgatccac acggagtact                                                  20


<210>  29
<211>  27
<212>  DNA
<213>  Artificial Sequence

<220>
<223>  Probe

<400>  29
atcaagatca ttgctcctcc tgagcgc                                          27


<210>  30
<211>  23
<212>  DNA
<213>  Artificial Sequence

<220>
<223>  Primer

<400>  30
cagcggtacc tctacacaga tga                                              23


<210>  31
<211>  19
<212>  DNA
<213>  Artificial Sequence

<220>
<223>  Primer

<400>  31
cgtcccatcc tccctgatc                                                   19


<210>  32
<211>  15
<212>  DNA
<213>  Artificial Sequence

<220>
<223>  Probe

<400>  32
cccagcagac agaag                                                       15


<210>  33
<211>  19
<212>  DNA
<213>  Artificial Sequence

<220>
<223>  Primer

<400>  33
cactattggc aacgagcgg                                                   19


<210>  34
<211>  21
<212>  DNA
<213>  Artificial Sequence

<220>
<223>  Primer

<400>  34
tccataccca agaaggaagg c                                                21


<210>  35
<211>  24
<212>  DNA
<213>  Artificial Sequence

<220>
<223>  Probe

<400>  35
tccgatgccc tgaggctctt ttcc                                             24

<210>  36
<211>  22
<212>  DNA
<213>  Artificial Sequence

<220>
<223>  Primer

<400>  36
gtttctttgc caacagccag at                                                22


<210>  37
<211>  22
<212>  DNA
<213>  Artificial Sequence

<220>
<223>  Primer

<400>  37
ccagcagcag ttctctgaag ct                                                22


<210>  38
<211>  18
<212>  DNA
<213>  Artificial Sequence

<220>
<223>  Probe

<400>  38
actttgatcc tgaggcct                                                     18

## Description

### Technical Field

The present invention relates to a substance capable of binding to at least a portion of a cis-element of the mRNA of FGF21 to inhibit the binding of a cis-element binding factor to the cis-element, and an agent for the prophylaxis or treatment of a lifestyle-related disease, which comprises the substance.

### (Background of the Invention)

Control of gene expression in cells is achieved by controlling the amount of mRNA transcribed, the stability of the transcribed mRNA, the amount translated into protein, and the stability of the protein itself at the respective stages of the transcription and translation of the mRNA. To date, a variety of methods of gene expression control that target these various control stages (antisense method, RNA interference and the like) have been developed. With the development of these methods, much attention has recently been drawn to the importance of the mechanism for expressional control mediated by the stability of mRNA and the amount translated into protein.

The stability of mRNA and the amount translated are controlled by a particular sequence of mRNA called "cis-element" and "a cis-element binding factor" that binds thereto.

A cis-element is defined as a region that is present in the 5' and 3' untranslated regions of DNA or RNA, and mediates the expressional control of the gene encoded by the DNA strand or RNA strand. "A cis-element binding factor" functions as a trans-acting factor that binds to a cis-element of a gene to promote or suppress the expression of the gene.

Cis-elements that are present in mRNA mediate the stability of the mRNA and the control of the amount translated into protein, serving as a key factor to determine the amount expressed of the gene product (protein) encoded by the mRNA.

A representative cis-element is the "AU-rich element (to be sometimes abbreviated as ARE in the present specification)". An ARE is a base sequence of about 10 to 150 bases that is rich in adenosine and uridine and abundantly present in the 3' untranslated region (3'-UTR) of mRNA. An ARE was first discovered as a region where the base sequence "AUUUA" frequently overlaps in the 3'-UTR of cytokines and lymphokines. AREs are currently estimated to be present in 5 to 8% of all genes; AREs are thought to be present in many genes mediating the maintenance of homeostasis (non-patent document 1).

An ARE-binding protein as a trans-acting factor binds to an ARE to promote or suppress the stability of mRNA and the amount translated (non-patent documents 2 and 3). Furthermore, micro-RNA (miRNA) also binds to ARE to achieve similar control (non-patent documents 4 and 5).

Patent Document 1 states that antisense oligonucleotides complementary to a portion of an ARE of the untranslated region on the 3' side of Bcl-2 mRNA (2'-O-(C1-C3)alkyl-oligonucleotides or 2'-O-methyl/deoxy-gapmers) are useful for the treatment/prophylaxis of apoptosis-related disease; specifically, a mixture of four oligonucleotides is bound to a sequence around the ARE to inhibit the instabilization of mRNA.

By the way, fibroblast growth factor 21 (to be sometimes abbreviated as FGF21 in the present specification) is known as a potent metabolic regulator (non-patent documents 6 - 8).
This factor is expressed selectively in the liver, regulating glucose uptake by adipocytes. It has been reported that administration of FGF21 to animal models of obesity and type 2 diabetes mellitus reduces glucose and triglyceride levels in the plasma and improves the levels of cardiovascular risk factors such as LDL-cholesterol reductions and HDL-cholesterol elevations. Furthermore, FGF21, unlike the other members of the FGF family, lacks mitogenic activity and does not cause hypoglycemia and body weight gain, and is therefore drawing much attention as a novel therapeutic drug for lifestyle-related disease with low prevalence of side effects.
However, no attempt has been made to improve the action of endogenous FGF21 by stabilizing the mRNA of FGF21 or controlling the translation level.

### [Document List]

### [patent document]

patent document 1: WO03/040182

### [non-patent documents]

non-patent document 1: Nucleic Acids Research, 2001, Vol.29, No. 1, p. 246-254
non-patent document 2: Nucleic Acids Research, 2005, Vol.33, No. 22, p. 7138-7150
non-patent document 3: Biochemical Society Transactions, 2002, Vol. 30, part 6, p. 952-958
non-patent document 4: Cell, 2005, Vol.120, No. 5, p. 623-634
non-patent document 5: Seminars in Cell and Developmental Biology, 2005, Vol. 16, No.1, p.49-58
non-patent document 6: Physiological Research, 2009, Vol.58, p. 1-7
non-patent document 7: Cellular and Molecular Life Science, 2009, Vol.66, p.2067-2073
non-patent document 8: BioDrugs, 2008, Vol.22, No. 1, p. 37-44

### [SUMMARY OF THE INVENTION]

### Problems to be Solved by the Invention

The present invention aims to provide an agent for the prophylaxis or treatment of a lifestyle-related disease. Means of Solving the Problems

The present inventors have conducted intensive studies in an attempt to solve the aforementioned problems and found a substance that binds to a cis-element of the mRNA of FGF21 and that inhibition of the binding of a cis-element binding factor to the cis-element can increase expression of FGF21 protein, which resulted in the completion of the present invention.

Accordingly, the present invention provides the following:
(1) A substance capable of binding to at least a portion of a cis-element of the mRNA of FGF21 to inhibit the binding of a cis-element binding factor to the cis-element.
(2) The substance described in (1) above, which is capable of binding to at least a portion of a cis-element of the mRNA of FGF21 to inhibit the binding of an AU-rich element binding factor to an AU-rich element.
(3) The substance described in (1) above, wherein the cis-element of the mRNA of FGF21 is the base sequence of the 835th to 842nd bases from the 5'-end side of the base sequence of the mRNA of FGF21 shown by SEQ ID NO:1.
(4) The substance described in (1) above, wherein the substance is a nucleic acid analogue, or a salt thereof, having a base sequence complementary to at least a portion of a cis-element of the mRNA of FGF21 and a base sequence complementary to a portion of the untranslated region on the 5' side and/or 3' side of the cis-element, and capable of inhibiting the binding of a cis-element binding factor to the cis-element by binding specifically to at least a portion of the cis-element on the basis of the sequence specificity possessed by the base sequence complementary to the untranslated region.
(5) The substance described in (1) above, wherein the substance is oligonucleotide, or a salt thereof, having a base sequence complementary to at least a portion of a cis-element of the mRNA of FGF21 and a base sequence complementary to a portion of the untranslated region on the 5' side and/or 3' side of the cis-element, and capable of inhibiting the binding of a cis-element binding factor to the cis-element by binding specifically to at least a portion of the cis-element on the basis of the sequence specificity possessed by the base sequence complementary to the untranslated region.
(6) The substance described in (5) above, wherein the substance comprises an LNA (locked nucleic acid).
(7) The substance described in (5) above, comprising a BNA (bridged nucleic acid).
(8) The substance described in (1) above, wherein the substance is an oligonucleotide containing a base sequence shown by SEQ ID NO:3, SEQ ID NO:18, SEQ ID NO:19, SEQ ID N0:20, SEQ ID NO:21, SEQ ID NO:22, SEQ ID NO:23 or SEQ ID NO:24 or substantially the same base sequence as the base sequence, or a salt thereof.
(9) The substance described in any one of (4) to (7) above, wherein the substance is an oligonucleotide containing a base sequence shown by SEQ ID NO:3 or substantially the same base sequence as the base sequence, or a salt thereof.
(10) The substance described in any one of (4) to (7) above, which is an oligonucleotide containing the base sequence shown by SEQ ID NO:19 or a base sequence substantially the same as the base sequence, or a salt thereof.
(11) The substance described in (1) above, which is an oligonucleotide represented by TUP#001, TUP#002, TUP#003, TUP#004, TUP#005, TUP#006, TUP#007, TUP#008, TUP#009, TUP#010, TUP#011, TUP#012, TUP#013, TUP#014, TUP#015, TUP#016, TUP#017, TUP#018, TUP#019, TUP#020, TUP#021, TUP#022, TUP#023, TUP#024, TUP#025, TUP#026, TUP#027, TUP#028, TUP#029, TUP#030, TUP#031, TUP#032 or TUP#033, or a salt thereof.
(12) A medicament comprising the substance described in (1) above.
(13) The medicament described in (12) above, which is an agent for the prophylaxis or treatment of a lifestyle-related disease.
(14) A method for the prophylaxis or treatment of a lifestyle-related disease in a mammal, comprising administering an effective amount of the substance described in (1) above to the mammal.
(15) Use of the substance described in (1) above for the production of an agent for the prophylaxis or treatment of a lifestyle-related disease.
(16) A carrier comprising the substance described in (5), (8), (9), (10) or (11) above.

(17) A medicament comprising a combination of two kinds or more of substances capable of binding to at least a portion of a cis-element of the mRNA that encodes each of two different kinds or more of target proteins to inhibit the binding of a cis-element binding factor to the cis-element.
(18) The medicament described in (17) above, wherein the substance is a substance capable of binding to at least a portion of a cis-element of the mRNA that encodes each target protein to inhibit the binding of an AU-rich element binding factor to the AU-rich element.
(19) The medicament described in (17) above, wherein the substance is a nucleic acid analogue, or a salt thereof, having a base sequence complementary to at least a portion of a cis-element of the mRNA that encodes each target protein and a base sequence complementary to a portion of the untranslated region on the 5' side and/or 3' side of the cis-element, and capable of inhibiting the binding of a cis-element binding factor to the cis-element by binding specifically to at least a portion of the cis-element on the basis of the sequence specificity possessed by the base sequence complementary to the untranslated region.
(20) The medicament described in (17) above, wherein the substance is an oligonucleotide, or a salt thereof, having a base sequence complementary to at least a portion of a cis-element of the mRNA that encodes each target protein and a base sequence complementary to a portion of the untranslated region on the 5' side and/or 3' side of the cis-element, and capable of inhibiting the binding of a cis-element binding factor to the cis-element by binding specifically to at least a portion of the cis-element on the basis of the sequence specificity possessed by the base sequence complementary to the untranslated region.
(21) The medicament described in (20) above, wherein the substance is an LNA-containing oligonucleotide or a salt thereof.
(22) The medicament described in (20) above, wherein the substance is a BNA-containing oligonucleotide or a salt thereof.

(23) An agent for the prophylaxis or treatment of a lifestyle-related disease, comprising a substance inhibiting the function of ZFP36L1 or ZFP36L2.
(24) A method for the prophylaxis or treatment of a lifestyle-related disease in a mammal, comprising administering an effective amount of the substance described in (23) above to the mammal.
(25) Use of the substance described in (23) above for the production of an agent for the prophylaxis or treatment of a lifestyle-related disease.

### Effect of the Invention

According to the present invention, it is possible to increase the expression of FGF21 protein to prevent/treat lifestyle-related disease by using a substance that binds to a cis-element of the mRNA of FGF21 to inhibit the binding of the cis-element and a cis-element binding factor.

### Brief Description of the Drawings

Figure 1 is a drawing showing a partial base sequence of the human FGF21 mRNA 3'-UTR and the base sequences of the oligonucleotides α-F21-A and α-F21-B designed with ARE-A and ARE-B as target cis-elements (Experimental Example 1).
Figure 2 is a drawing showing the results of a measurement of the amount of FGF21 mRNA in an oligonucleotide-incorporating cell line of human liver derivation (HepG2) by a real time PCR method (Experimental Example 1).
Figure 3 is a drawing showing the results of a measurement of the amount of FGF21 mRNA in an oligonucleotide-incorporating cell line of mouse liver derivation (H2.35) by a real time PCR method (Experimental Example 1).
Figure 4 is a drawing showing the effect of WY-14643 in amplifying the amount of intracellularly transcribed FGF21 mRNA in mouse hepatocytes (H2.35) and the intracellular instability of FGF21 mRNA with the inhibition of the transcription by actinomycin D treatment (Experimental Example 2).
Figure 5 is a drawing showing the results of an investigation of the effect of introduction of an oligonucleotide on the amount of FGF21 mRNA in the absence of WY-14643 in HepG2 cells (Experimental Example 2).
Figure 6 is a drawing showing the results of an investigation of the effect of introduction of an oligonucleotide on the amount of FGF21 mRNA in the presence of WY-14643 in H2.35 cells (Experimental Example 2).
Figure 7 is a drawing showing the results of a measurement of the amount of FGF21 protein secreted in H2.35 cells by an ELISA method (Experimental Example 2).
Figure 8 is a drawing showing MS spectral data on ZFP36L1 (A) and ZFP36L2 (B), both identified with FGF21-3'UTR as a bait (decoy) (Experimental Example 3).
Figure 9 is a drawing showing the results of an evaluation of the capacity of ZFP36L1 for binding to the FGF21 mRNA 3'UTR and the inhibition of the binding by α-F21-A (LNA) and α-F21-B (LNA) by Western blot analysis (Experimental Example 4).
Figure 10 is a drawing showing the results of a measurement of the amount of FGF21 mRNA by a real time PCR method when the expression of ZFP36L1 and ZFP36L2 is suppressed using RNAi (RNA interference) (Experimental Example 5) .
Figure 11 is a schematic diagram explaining the mechanism for the enhancement of the expression of FGF21 by an oligonucleotide relating to the present invention.
Figure 12 is a drawing showing the positional relationships between TUP#003 to TUP#033 and FGF21 mRNA and the positions of LNA-modifying bases in TUP#003 to TUP#033. In the drawing, "FGF21 mRNA" depicts a portion of the 3'UTR of the mRNA (the partial base sequence of the 826th to 843rd bases from the 5' end of the mRNA; SEQ ID NO:25) in the 3'→5' direction.
Figure 13 is a drawing showing the results of a measurement of the amount of FGF21 mRNA in oligonucleotide-incorporating cell line Hep3B of human liver derivation by a real time PCR method (Experimental Example 6).
Figure 14 is a drawing showing the results of a measurement of the amount of FGF21 mRNA in the primary culture of oligonucleotide-incorporating mouse liver cell by a real time PCR method (Experimental Example 7).
Figure 15 is a drawing showing the results of a measurement of the amount of FGF21 protein secreted from oligonucleotide-incorporating cell line Hep3B of human liver derivation by ELISA (Experimental Example 8).
Figure 16 is a drawing showing the results of a measurement of the amount of FGF21 protein secreted from oligonucleotide-incorporating mouse liver primary culture cell by ELISA (Experimental Example 9).
Figure 17 is a drawing showing the results of a measurement of the amount of FGF21 mRNA in a mouse liver after administration of an oligonucleotide (TUP#007) by a real time PCR method (Experimental Example 10).

### (Detailed Description of the Invention)

The present invention relates to a substance capable of binding to at least a portion of a cis-element of the mRNA of FGF21 (fibroblast growth factor 21) to inhibit the binding of a cis-element binding factor to the cis-element (hereinafter to be sometimes abbreviated as "the substance of the present invention").

Examples of preferable mRNAs of FGF21, i.e., mRNAs that encode the protein of FGF21, include, for example, the mRNA of human FGF21 (to be sometimes abbreviated as "mRNA of FGF21 shown by SEQ ID NO: 1" in the present specification) shown by the base sequence obtained by reading "t" as "u" in human FGF21 cDNA (base sequence shown by SEQ ID NO: 1, GenBank Accession No. NM_019113), or homologues thereof in mammals other than humans (e.g., mice, rats, rabbits, sheep, swine, bovines, cats, dogs, monkeys) (for example, the mRNA of mouse FGF21 shown by the base sequence obtained by reading "t" as "u" in the mouse FGF21 cDNA (GenBank Accession No. NM_020013) consisting of the base sequence shown by SEQ ID NO:9), as well as naturally occurring allelic mutants thereof and the like.

Herein, "a cis-element" is a region that is present on the same mRNA (i.e., upstream or downstream of the translated region), mediates the stability of the mRNA and the control of the amount translated into protein, and serves as a key factor to determine the amount expressed of the gene product (protein) encoded by the mRNA. Specific examples of the cis-element include AU-rich element, Histone mRNA 3'-UTR stem loop element, Internal ribosome entry site (IRES), A2RE element, ZIPCODE element, Iron response element (IRE), Cytoplasmic polyadenylation (CPE), Nanos translational control, Amyloid precursor protein element (APP), Translational regulation element (TGE)/direct repeat element (DRE), Bruno element(BRE), 15-lipoxygenase differentiation control element (15-LOX-DICE), G-quartet element, Adh mRNA down-regulation element, Barley yellow dwarf virus element, GLUT1 mRNA-stability control element, Msl-2 3'-UTR control element, Msl-2 5'-UTR control element, Ribosomal S12 mRNA translational control element, Selenocysteine insertion sequence type 1 (SECIS-1), Selenocysteine insertion sequence type 2 (SECIS-2), TNF-mRNA stability control element, Terminal oligopyrimidine tract (TOP), Vimentin mRNA 3'-UTR control element and the like. As a particularly preferable example of the above-mentioned cis-element, AU-rich element can be mentioned.

An AU-rich element (ARE) is a base sequence of about 10 to 150 bases that is rich in adenosine and uridine and abundantly present in the 3'-UTR (untranslated region) of mRNA, mediating the stability of mRNA and the control of translation level. AREs are currently tentatively classified into (1) regions comprising several copies of the "AUUUA pentamer" in an uridine-rich sequence (ARE I), (2) regions comprising at least two or more overlapping "UUAUUUA(U/A)(U/A) nonamers" (ARE II), and (3) regions that do not comprise the "AUUUA pentamer" but are rich in uridine (ARE III). In the present invention, AU-rich elements are understood to include at least these three groups of AREs.

Specific examples of the AU-rich element include the base sequence of the 814th to 821st bases from the 5' end side of the base sequence of the mRNA of FGF21 shown by SEQ ID NO:1 (to be sometimes indicated as ARE-A) and the base sequence of the 835th to 842nd bases from the 5' end side of the base sequence of the mRNA of FGF21 shown by SEQ ID NO:1 (to be sometimes indicated as ARE-B). Examples of preferable AU-rich elements include ARE-B.

Herein, "a cis-element binding factor" means a factor having the function of binding to a cis-element to improve or reduce the stability of the mRNA containing the cis-element and promote or suppress the expression of the protein encoded by the mRNA containing the cis-element.

Examples of the cis-element in the above-described "cis-element-binding factor" include the aforementioned cis-elements. Preferable examples of the cis-elements include AU-rich elements.

Examples of the binding factor in "a cis-element-binding factor" as mentioned herein include proteins and miRNAs (micro RNAs) having the function of binding to the cis-element to improve or reduce the stability of the mRNA containing the cis-element and promote or suppress the expression of the protein encoded by the mRNA containing the cis-element. Preferable examples of the binding factor include proteins and miRNAs having the function of binding to the cis-element to reduce the stability of the mRNA containing the cis-element and suppress the expression of the protein encoded by the mRNA containing the cis-element.
Examples of more preferable binding factors as described above include proteins having the function of binding to the cis-element to reduce the stability of the mRNA containing the cis-element and suppress the expression of the protein encoded by the mRNA containing the cis-element.

Preferable examples of the "cis-element-binding factor" include AU-rich-element binding factors. An AU-rich-element binding factor means a factor having the function of binding to an AU-rich element to improve or reduce the stability of the mRNA containing the AU-rich element and promote or suppress the expression of the protein encoded by the mRNA containing the cis-element. Specific examples of proteins that are AU-rich-element binding factors having the function of binding to an AU-rich element to improve or reduce the stability of the mRNA containing the AU-rich element and promote or suppress the expression of the protein encoded by the mRNA containing the cis-element include AUF1, HuR, Hel-N1, Hud, TTP, BRF1, TIA-1, KSRP, GUG-BP2, Nucleotin, TINO, PAIP2, ZFP36L1 and ZFP36L2; specific examples of miRNAs include miR16.

Examples of preferable AU-rich-element binding factors include proteins and miRNAs having the function of binding to the AU-rich element to reduce the stability of the mRNA containing the AU-rich element and suppress the expression of the protein encoded by the mRNA containing the cis-element. More preferable examples of the binding factor include proteins having the function of binding to the AU-rich element to reduce the stability of the mRNA containing the AU-rich element and suppress the expression of the protein encoded by the mRNA containing the cis-element. Specific examples of AU-rich-element binding factors having the function of reducing the stability of the mRNA containing an AU-rich element and suppress the expression of the protein encoded by the mRNA containing the cis-element include ZFP36L1 and ZFP36L2. Examples of preferable AU-rich-element binding factors having the function of reducing the stability of the mRNA containing an AU-rich element and suppress the expression of the protein encoded by the mRNA containing the cis-element include human ZFP36L1 (GenBank Accession No. NM_004926.2) and human ZFP36L2 (GenBank Accession No. NM_006887.4).

The substance of the present invention selectively stabilizes the mRNA of FGF21 to promote the expression of the FGF21 protein encoded by the mRNA of FGF21, i.e., the translation of FGF21, by inhibiting the binding of a cis-element binding factor to a cis-element of the mRNA of FGF21.

The substance of the present invention is exemplified by nucleic acid analogues (e.g., oligonucleotides (naturally occurring oligonucleotides and artificial analogues thereof), miRNAs and mimics thereof)) (to be sometimes referred to as "the oligonucleotide of the present invention" in the present specification) or salts thereof, low molecular compounds (e.g., non-peptide compounds, peptides) or salts thereof and the like.

Specific examples of the nucleic acid analogue include nucleic acid analogues having a base sequence complementary to at least a portion of a cis-element of the mRNA of FGF21 and a base sequence complementary to a portion of the untranslated region on the 5' side and/or 3' side of the cis-element, and capable of inhibiting the binding of a cis-element binding factor to the cis-element by binding specifically to at least a portion of the cis-element, on the basis of the sequence specificity possessed by the base sequence complementary to the untranslated region. Preferable examples of the nucleic acid analogue include oligonucleotides or salts thereof.

In the present invention, "a base sequence complementary to a portion" refers to a base sequence that is complementary with respect to a base length sufficient to significantly stabilize the target mRNA and promote the translation when hybridized with the target cis-element sequence. When the cis-element is an AU-rich element which is "the UUAUUUA(U/A)(U/A) nonamer", examples of "a base sequence complementary to a portion" include base sequences complementary with respect to one continuous base or more in the nonamer; preferable examples include base sequences complementary with respect to five continuous bases or more in the nonamer; more preferable examples include base sequences complementary with respect to six continuous bases or more in the nonamer.
"A base sequence complementary to a portion of the untranslated region on the 5' side and/or 3' side of a cis-element" may be any one complementary to a portion of the untranslated region on the 5' side and/or 3' side of the target cis-element with respect to a base length sufficient to confer the capacity of binding specifically to a cis-element of a particular mRNA to the nucleic acid analogue. When the cis-element is an AU-rich element (ARE) of the mRNA of human FGF21, examples of a base sequence complementary to a portion of the untranslated region on the 5' side and/or 3' side of a cis-element include base sequences complementary with respect to one base or more as counted for the sum on the 5' side and 3' side of the ARE; preferable examples include base sequences complementary with respect to 3 to 9 bases as counted for the sum on the 5' side and 3' side of the ARE. Examples of still more preferable base sequences complementary to a portion of the untranslated region on the 5' side and/or 3' side of a cis-element include base sequences complementary with respect to 3 to 9 bases as counted for the sum on the 5' side and 3' side of ARE-B.
Specific examples of the above-described base sequence complementary to a portion of the untranslated region on the 5' side and/or 3' side of a cis-element include base sequences complementary to the base sequence of the 828th to 834th bases from the 5' end side of the base sequence of the mRNA of FGF21 shown by SEQ ID NO:1, base sequences complementary to the base sequence of the 826th to 834th bases, base sequences complementary to the base sequence of the 832nd to 834th bases, and base sequences complementary to the base sequence of the 830th to 834th bases.
Preferable examples of the above-described base sequence complementary to a portion of the untranslated region on the 5' side and/or 3' side of a cis-element include base sequences complementary to the base sequence of the 828th to 834th bases from the 5' end side of the base sequence of the mRNA of human FGF21, and base sequences complementary to the base sequence of the 826th to 834th bases. More preferable examples of the above-described base sequence complementary to a portion of the untranslated region on the 5' side and/or 3' side of a cis-element include base sequences complementary to the base sequence of the 826th to 834th bases from the 5' end side of the base sequence of the mRNA of human FGF21.

The "oligonucleotide" of the present invention is a single-stranded nucleic acid capable of binding specifically to the mRNA of FGF21 to stabilize the mRNA by having a base sequence complementary to at least a portion of a cis-element of the mRNA of FGF21 and a base sequence complementary to a portion of the untranslated region on the 5' side and/or 3' side of the cis-element, by which stabilization it is possible to raise the level of the translation of FGF21 protein from the mRNA of FGF21.

Examples of the oligonucleotide of the present invention include polydeoxyribonucleotide containing 2-deoxy-D-ribose, polyribonucleotide containing D-ribose, other types of polynucleotide that is N-glycoside of purine or pyrimidine base, or another polymer having a non-nucleotide backbone (for example, commercially available protein nucleic acids and synthetic sequence specific nucleic acid polymers) or another polymer having a special bond (however, this polymer comprises a nucleotide having a configuration that allows base pairing or base attachment as found in DNA and RNA) or the like. These may be those having a known modification added thereto, for example, those with a label known in the relevant field, those with a cap, those methylated, those having 1 or more naturally occurring nucleotides substituted by analogues, those modified with an intramolecular nucleotide, for example, those having a non-charge bond (for example, methylphosphonate, phospho triester, phosphoramidate, carbamate), those having a charged bond or a sulfur-containing bond (for example, phosphorothioate, phosphorodithioate), for example, those having a side chain group of a protein (for example, nuclease inhibitor, toxin, antibody, signal peptide, poly-L-lysine) or a sugar (for example, monosaccharide), those having an intercalating compound (for example, acridine, psoralen), those containing a chelate compound (for example, metals, radioactive metals, boron, oxidizing metals), or those containing an alkylating agent, or those having a modified bond (for example, α-anomer type nucleic acid). Here, "nucleoside", "nucleotide" and "oligonucleotide" may include not only those containing the purine and pyrimidine bases, but also those containing another modified heterocycle type base. These modified products may contain a methylated purine and pyrimidine, an acylated purine and pyrimidine, or another heterocycle. The modified nucleoside and modified nucleotide may also have a modified saccharide moiety and, for example, one or more hydroxyl groups may be substituted by halogen, aliphatic group and the like, or may be converted to a functional group such as ether, amine and the like.

The length of the oligonucleotide of the present invention is not particularly limited, as far as the oligonucleotide is capable of hybridizing specifically with a sequence comprising at least a portion of a cis-element of the mRNA of FGF21 to thereby promote the translation into FGF21 protein; the length is about 8 bases for the shortest and about 40 bases for the longest, preferably about 12 to about 20 bases, particularly preferably 12 to 18 bases.
A big feature of the present invention resides in the fact that by narrowing the target region of ARE element to a narrower region, and synthesizing an oligonucleotide with LNA, BNA and the like, the expression of FGF21 can be controlled with, for example, a very short antisense oligonucleotide of about 12-mer (12 - 18 mer) length (even with this base length, high affinity and selectivity for target mRNA are retained).

Although the nucleotide molecule that constitutes the oligonucleotide of the present invention may be a natural type DNA or RNA, the molecule may be a nucleotide molecule undergoing various chemical modifications in order to increase the stability (chemical and/or to-enzyme) or specific activity (affinity for RNA), and may also be a nucleic acid analogue. Examples of the above-described chemical modifications include modifications to replace the phosphoric acid residue (phosphate) of each nucleotide constituting the oligonucleotide with a chemically modified phosphoric acid residue such as phosphorothioate (PS), methylphosphonate, phosphorodithionate, or boranophosphate.
Other examples of the above-described chemical modifications include modifications to replace the 2'-position hydroxyl group of the sugar of each nucleotide with another functional group. Here, other functional groups include modifications to replace the hydroxyl group with a halogen atom (e.g., fluorine atoms); a C₁₋₆ alkyl group (e.g., methyl group); an amino group that may be substituted by a C₁₋₆ alkyl group (e.g., methyl group); or -OR (R is, for example, CH₃(2'-O-Me), CH₂CH₂OCH₃(2' -O-MOE), CH₂CH₂NHC (NH)NH₂, CH₂CONHCH₃, CH₂CH₂CN) . As still another modification in addition to the above-described chemical modifications, the base moiety (pyrimidine, purine) may be chemically modified. Examples of chemical modifications to be made to the base moiety include modifications to introduce a methyl group or a cationic functional group into the 5-position of the pyrimidine base, or modifications to replace the 2-position carbonyl group with a thiocarbonyl group.
Examples of the nucleic acid analogue include UNA (unlocked nucleic acid), HNA, morpholino oligo, and PNA (peptide nucleic acid). The HNA may have the hydroxyl group in the hexopyranose moiety thereof deoxidated. The HNA may have the hydroxyl group in the hexopyranose moiety thereof replaced by a fluorine atom.

Regarding the conformation of the sugar moiety of RNA, two types are dominant: C2'-endo (S type) and C3'-endo (N type); in a single-stranded RNA, the conformation occurs as an equilibrium between the two, but when a double strand is formed, the conformation is fixed at the N type. Therefore, BNA, LNA (Imanishi, T. et al., Chem. Commun., 1653-9, 2002; Jepsen, J.S. et al., Oligonucleotides, 14, 130-46, 2004), ENA (Morita, K. et al., Nucleosides Nucleotides Nucleic Acids, 22, 1619-21, 2003), cEt, cMOE (Seth et al., Journal of Medicinal Chemistry, 51, 10-13, 2009) and the like, which are RNA derivatives wherein the conformation of the sugar moiety is fixed at the N type by bridging the 2' oxygen and 4' carbon to confer a strong bindability to target RNA, can also be preferably used as oligonucleotide-constituting nucleotide molecules.
As BNA and LNA, specifically, ones described in WO2005/021570, WO03/068695 or WO2001/007455 can be used; the oligonucleotide of the present invention may contain these BNA and/or LNA.

When the nucleotide molecule constituting the oligonucleotide of the present invention is a chemically modified nucleotide molecule, specific examples of the oligonucleotide of the present invention include oligonucleotides resulting from replacement of some or all of the constituent nucleotides of an oligonucleotide (e.g., SEQ ID NO:2, SEQ ID NO: 3, SEQ ID NO: 18, SEQ ID NO: 19, SEQ ID NO: 20, SEQ ID NO: 21, SEQ ID NO: 22, SEQ ID NO: 23, SEQ ID NO: 24) with a BNA (e.g., 2',4'-BNA^{NC}, 2',4'-BNA^{COC}) or LNA (2',4'-BNA) described in the pamphlet for WO2005/021570, WO03/068695 or WO2001/007455, or S-oligo-modified oligonucleotides (oligonucleotides resulting from replacement of phosphate residues among the various nucleotides constituting an oligonucleotide with chemically modified phosphate residues of phosphorothioate). More specifically, oligonucleotides of which some or all of the constituent nucleotides are synthesized using the compound 5 described in Example 1 (BNA^{NC}-adenosine monomer), the compound 9 described in Example 2 (BNA^{NC}-guanosine monomer), or the BNA^{NC}-pyrimidine monomer that can be synthesized in the same way, can be mentioned.
The aforementioned oligonucleotides resulting from replacement of phosphate residues among the various nucleotides constituting an oligonucleotide with chemically modified phosphate residues of phosphorothioate are oligonucleotides wherein phosphate residues among the various nucleotides constituting the oligonucleotide have undergone the chemical modification shown by Formula 1.

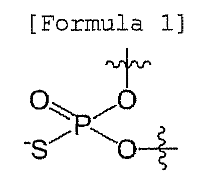

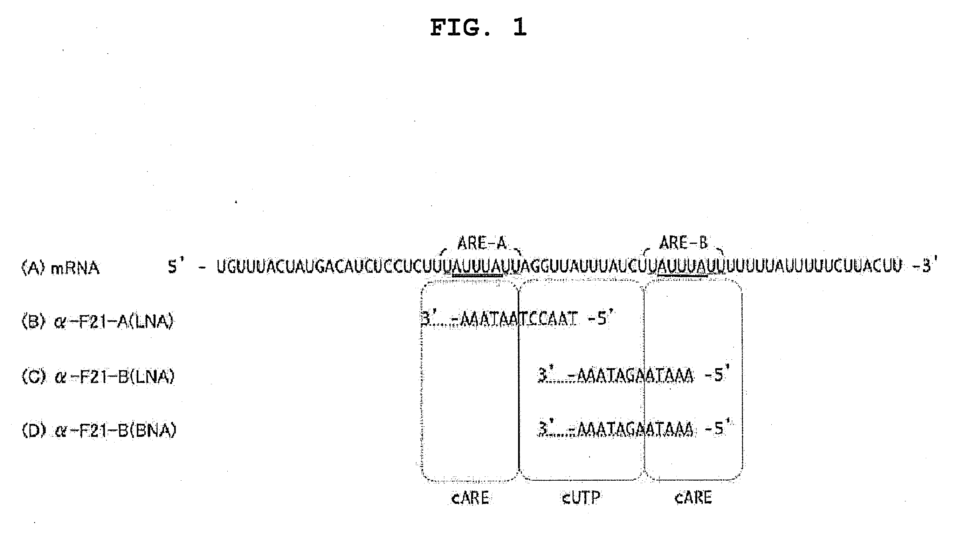

The oligonucleotide of the present invention is preferably one capable of binding to at least a portion of an AU-rich element (ARE-A or ARE-B, preferably ARE-B) of the mRNA of human FGF21.
As shown in Figure 1, portions of ARE-A (the base sequence of the 814th to 821st bases from the 5' end side of ) the base sequence of the mRNA of human FGF21) include the base sequence of the 816th to 821st bases from the 5' end side of the base sequence of the mRNA of human FGF21 and the like.
Examples of a portion of ARE-B (the base sequence of the 835th to 842nd bases from the 5'-end side of the base sequence of the mRNA of human FGF21) include the base sequence of the 835th to 839th bases from the 5'-end side of the base sequence of the mRNA of human FGF21, the base sequence of the 835th to 841st bases, the base sequence of the 835th to 837th bases and the like. Examples of preferable portions of ARE-B (the base sequence of the 835th to 842nd bases from the 5'-end side of the base sequence of the mRNA of human FGF21) include the base sequence of the 835th to 839th bases and the base sequence of the 835th to 841st bases. Examples of more preferable portions of ARE-B (the base sequence of the 835th to 842nd bases from the 5' end side of the base sequence of the mRNA of human FGF21) include the base sequence of the 835th to 841st bases.
With respect to the oligonucleotide capable of binding to at least a portion of ARE-A, the length is not particularly limited as stated above; for example, an oligonucleotide having a base sequence complementary to the base sequence of the 816th to 827th bases from the 5'-end side of the base sequence of the mRNA of human FGF21 (SEQ ID NO:2) and the like are used.
With respect to the oligonucleotide capable of binding to at least a portion of ARE-B, the length is not particularly limited as stated above; examples include an oligonucleotide having a base sequence complementary to the base sequence of the 828th to 839th bases from the 5'-end side of the base sequence of the mRNA of human FGF21 (SEQ ID NO:3), an oligonucleotide having a base sequence complementary to the base sequence of the 826th to 843rd bases (SEQ ID NO:18), an oligonucleotide having a base sequence complementary to the base sequence of the 826th to 841st bases (SEQ ID NO:19), an oligonucleotide having a base sequence complementary to the base sequence of the 828th to 841st bases (SEQ ID NO:20), an oligonucleotide having a base sequence complementary to the base sequence of the 826th to 839th bases (SEQ ID NO:21), an oligonucleotide having a base sequence complementary to the base sequence of the 832nd to 843rd bases (SEQ ID NO:22), an oligonucleotide having a base sequence complementary to the base sequence of the 830th to 841st bases (SEQ ID NO:23), and an oligonucleotide having a base sequence complementary to the base sequence of the 826th to 837th bases (SEQ ID NO:24).
Out of those mentioned above, oligonucleotides having a base sequence shown by SEQ ID NO:3, SEQ ID NO:18, SEQ ID NO:19, SEQ ID NO:20, SEQ ID NO:21, SEQ ID NO:22, SEQ ID NO:23 or SEQ ID NO:24 are preferable, with greater preference given to oligonucleotides having a base sequence shown by SEQ ID NO:3 or SEQ ID NO:19.

Specific preferable examples of the oligonucleotide of the present invention are TUP#001 to TUP#033 shown in Table 1 below (of these, TUP#001 is sometimes indicated as α-F21-B(BNA), and TUP#002 as α-F21-B(LNA)), more preferable examples include TUP#001, TUP#002, TUP#003, TUP#004, TUP#005, TUP#006, TUP#007, TUP#008, TUP#011, TUP#015, TUP#016, TUP#019, TUP#023 and TUP#030. Still more preferable examples of the oligonucleotide of the present invention include TUP#003, TUP#004, TUP#007, TUP#015 and TUP#019. Particularly preferable example of the oligonucleotide of the present invention is TUP#007.

The oligonucleotide of the present invention may be an oligonucleotide having substantially the same base sequence as the base sequence shown by SEQ ID NO:2, SEQ ID NO: 3, SEQ ID NO: 18, SEQ ID NO: 19, SEQ ID NO: 20, SEQ ID NO: 21, SEQ ID NO: 22, SEQ ID NO: 23 or SEQ ID NO: 24.

Here, "an oligonucleotide having substantially the same base sequence" means an oligonucleotide that hybridizes specifically with a portion of ARE-A or ARE-B of the mRNA of FGF21, and is capable of promoting the translation of FGF21 protein from the mRNA, under physiological conditions in cells, specifically exemplified by oligonucleotides having a base sequence possessing an identity of 80% or more, preferably 90% or more, to the base sequence shown by each sequence identification number shown above, or oligonucleotides having a base sequence resulting from substitution of one or two bases with other bases in the base sequence shown by each sequence identification number shown above.

The oligonucleotide of the present invention, including the above-described oligonucleotides containing various modifications, can all be chemically synthesized by a method known per se described in WO2005/021570, WO03/06869 or WO2001/007455 or a method based thereon. Desired modified oligonucleotides are available by consignment synthesis (e.g., Gene Design Inc.).

The oligonucleotide of the present invention may be supplied in a special form like liposome or microspheres, and can be given in an adduct form of a modification group. As such a specific form and an adduct form used, a polycation like polylysine, which acts to neutralize the charge of the phosphate backbone, and a hydrophobic compound like a lipid that enhances the interaction with cell membrane or increases nucleic acid uptake (for example, phospholipid, cholesterol and the like) can be mentioned. As lipids preferred for addition, cholesterol and derivatives thereof (for example, cholesterylchloroformate, cholic acid) can be mentioned. These can be attached to the 3' end or the 5' end of nucleic acid, and can be attached via a base, a sugar or an intramolecular nucleoside bond. As other groups, a capping group specifically arranged at the 3' end or 5' end of nucleic acid to prevent degradation by a nuclease such as exonuclease or RNase can be mentioned. As such a capping group, hydroxyl group protecting groups known in the relevant field, including glycols such as polyethylene glycol and tetraethylene glycol can be mentioned, which, however, are not to be construed as limiting.

The oligonucleotide of the present invention may form a salt with an inorganic base, an organic base, an inorganic acid, an organic acid and the like. Examples of the above-mentioned salt with an inorganic base include alkali metal salts such as sodium salt and potassium salt; alkaline earth metal salts such as calcium salt, magnesium salt and the like; and aluminum salt, ammonium salt and the like. Examples of the above-mentioned salt with an organic base include salts with trimethylamine, triethylamine, pyridine, picoline, ethanolamine, diethanolamine, triethanolamine, dicyclohexylamine and N,N'-dibenzylethylenediamine. Examples of the above-mentioned salt with an inorganic acid include salts with hydrochloric acid, hydrobromic acid, nitric acid, sulfuric acid and phosphoric acid. Examples of the above-mentioned salt with an organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid and p-toluenesulfonic acid. Among these salts, a pharmacologically acceptable salt is preferable, and a sodium salt is more preferable.
The oligonucleotide of the present invention may form a complex with an oligonucleotide having a sequence complementary to the nucleotide.

The substance of the present invention is not limited to nucleic acid analogues comprising a base sequence complementary to at least a portion of a cis-element of the mRNA of FGF21 as described above, and may be a low molecular compound or a salt thereof or the like, as far as it promotes the production of FGF21 protein via stabilization of the mRNA by binding to the cis-element. Although a low molecular compound that binds to a cis-element of the mRNA of FGF21 to promote the translation of FGF21 protein from the mRNA can be acquired by, for example, determining the capacity of binding between a nucleic acid having the same base sequence as the cis-element and a test compound via labeling either the nucleic acid or the test compound, and further evaluating the stabilization of the mRNA of FGF21 or the increase in the amount of FGF21 protein produced in the presence of the test compound using, for example, RT-PCR or a variety of immunological analyses, any other screening methods known per se can be utilized.

The FGF21 protein expression promoting activity possessed by the substance of the present invention can be examined using a transformant incorporating the FGF21 gene, an in vivo or ex vivo FGF21 gene expression system, or an in vivo or ex vivo FGF21 protein expression system.

As a salt of a low-molecular-weight compound, while any salt may be as long as it is a nontoxic salt, for example, a salt with a pharmacologically acceptable acid (e.g., inorganic acid, organic acid), a base (e.g., alkali metal, alkaline earth metal) and the like are used, and a pharmacologically acceptable acid addition salt is particularly preferable. Useful salts include, for example, salts with inorganic acids (for example, hydrochloric acid, phosphoric acid, hydrobromic acid, sulfuric acid and the like) or salts with organic acids (for example, acetic acid, formic acid, propionic acid, fumaric acid, maleic acid, succinic acid, tartaric acid, citric acid, malic acid, oxalic acid, benzoic acid, ) methanesulfonic acid, benzenesulfonic acid and the like) and the like.

In addition, the present invention relates to a medicament comprising the aforementioned substance of the present invention (to be sometimes referred to as "the medicament of the present invention" in the present specification).

As mentioned above, since an effect of FGF21 for the prophylaxis or treatment of lifestyle-related diseases such as diabetes, obesity and the like has been reported, the medicament of the present invention can be used as an agent for the prophylaxis or treatment of the lifestyle-related diseases.

Specific examples of the lifestyle-related diseases that can be prevented or treated by the medicament of the present invention include diabetes, cerebral apoplexy, cerebral hemorrhage, cerebral infarction, cardiac disease (e.g., myocardial infarction, angina pectoris), hyperlipidemia, hypertension, obesity, metabolic syndrome, chronic bronchitis, COPD (chronic obstructive pulmonary disease), emphysema, lung squamous cell carcinoma, colorectal cancer, alcoholic hepatitis, gout and the like. Of these, diabetes and obesity are preferable.

The oligonucleotide of the present invention can be contained in a medicament by being bound to or enclosed in a carrier.

Examples of carriers for binding and including the oligonucleotide of the present invention include liposomes and lipoplexes. In particular, to make the substance of the present invention easily transportable into cells, liposomes are preferable. Preferable liposomes include positively charged liposomes, positively charged cholesterols, and membrane-permeable-peptide binding liposomes (Mamoru Nakanishi et al., Protein, Nucleic Acid and Enzyme, 44: 1590-1596 (1999), Shiroh Futaki, Kagaku To Seibutsu, 43: 649-653 (2005), Clinical Cancer Research 59: 4325-4333 (1999) and the like).

The oligonucleotide of the present invention, capable of complementarily binding to a transcription product of the FGF21 gene to raise the translation level of the transcription product, promotes the function and action of FGF21 protein in living organisms, and can be used as a prophylactic/therapeutic agent for lifestyle-related disease.

The substance of the present invention or the medicament of the present invention is of low toxicity, and can be orally or non-orally administered (e.g., intravascular administration, subcutaneous administration, rectal administration, transurethral administration, intraperitoneal administration) as it is, or as an appropriate dosage form of pharmaceutical composition, to mammals (e.g., mice, rats, rabbits, sheep, swine, bovines, cats, dogs, monkeys, humans).

When the oligonucleotide of the present invention is used as the above-described prophylactic/therapeutic agent for lifestyle-related disease, the same can be prepared and administered as pharmaceutical formulations according to a method known per se. The oligonucleotide can be administered as it is, or along with an auxiliary for promoting its ingestion, using a gene gun or a catheter such as a hydrogel catheter. Alternatively, the oligonucleotide can be prepared as an aerosol and topically administered into the trachea as an inhalant.

Furthermore, for the purpose of improving the disposition, extending the half-life, and increasing the intracellular uptake efficiency, the aforementioned oligonucleotide may be prepared as a preparation (injection) alone or with a carrier such as a liposome, and administered venously, subcutaneously and the like.

The medicament of the present invention may contain a pharmacologically acceptable carrier, diluent or excipient and the like. The medicament of the present invention is supplied in the form of a dosage form suitable for oral or non-oral administration.

When the medicament of the present invention is non-orally administered, the same is administered as, for example, injections or suppositories. The injections may include dosage forms such as intravenous injections, subcutaneous injections, intracutaneous injections, intramuscular injections and drip infusion injections. Such an injection can be prepared according to a publicly known method. An injection can be prepared by, for example, dissolving, suspending or emulsifying the above-described substance of the present invention in a sterile aqueous or oily solution in common use for injections. Aqueous solutions for injection include, for example, physiological saline and an isotonic solution containing glucose or another auxiliary drug, which may be used in combination with an appropriate solubilizer, for example, alcohol (e.g., ethanol), polyalcohol (e.g., propylene glycol, polyethylene glycol), a non-ionic surfactant [e.g., polysorbate 80, HCO-50 (polyoxyethylene (50 mol) adduct of hydrogenated castor oil)]. Such oily solutions include, for example, sesame oil and soybean oil, which may be used in combination with benzyl benzoate, benzyl alcohol and the like as solubilizers. The prepared injection liquid is preferably filled in an appropriate ampoule. Such suppositories can be prepared according to a publicly known method by, for example, mixing the above-described nucleic acid in an ordinary suppository base.

When the medicament of the present invention is orally administered, it is administered as solid or liquid dosage forms, specifically tablets (including sugar-coated tablets and film-coated tablets), pills, granules, powders, capsules (including soft capsules), syrups, emulsions, suspensions and the like. The medicament of the present invention is produced by a publicly known method, and may contain a carrier, diluent or excipient in common use in the field of medicament making. When the medicament of the present invention is a tablet, the tablet may contain carriers and excipients for tablets (for example, lactose, starch, sucrose, magnesium stearate).

The medicament of the present invention is conveniently prepared in a medication unit suitable for the dosage of the active ingredient. Dosage forms prepared in a medication unit suitable for the dosage of the active ingredient include, for example, tablets, pills, capsules, injections (ampoules), and suppositories. It is preferable that the substance of the present invention be contained normally 5 to 500 mg, for example, particularly 5 to 100 mg for injections or 10 to 250 mg for other dosage forms, per medication unit dosage form.

The dose of the medicament of the present invention varies depending on the recipient of administration, target disease, symptoms, route for administration and the like; for example, when the medicament is used for the treatment/prevention of adult diabetes mellitus or obesity, it is convenient to administer the nucleic acid of the present invention usually at about 0.01 to 20 mg/kg body weight, preferably about 0.1 to 10 mg/kg body weight, more preferably about 0.1 to 5 mg/kg body weight, based on a single dose, about 1 to 5 times a day, preferably about 1 to 3 times a day, by venous injection. In the case of other modes of non-oral administration and oral administration, similar doses may be administered. In case the symptom is particularly severe, the dose may be increased according to the symptom.

The medicament of the present invention may comprise another active ingredient, as far as it does not have an unwanted interaction when blended with the substance of the present invention.

Furthermore, the medicament of the present invention may be used in combination with other drugs, for example, antidiabetic drugs such as insulin resistance ameliorators (e.g., thiazolidine derivatives such as rosiglitazone and pioglitazone), hypoglycemic drugs (e.g., sulfonylurea drugs such as glibenclamide, glimepiride, tolbutamide, glycopyramide, and acetohexamide, sulfonamide drugs such as glymidine and glybuzole, biguanide drugs such as metformin and buformin), aldose reductase inhibitors (e.g., epalrestat), α-glucosidase inhibitors (e.g., voglibose, acarbose), and somatomedin C preparations (e.g., mecasermin); and antiobesity drugs such as centrally acting antiobesity drugs (e.g., dexfenfluramine, fenfluramine, phentermine), MCH receptor antagonists (e.g., SB-568849, SNAP-7941), neuropeptide Y antagonists (e.g., CP-422935), cannabinoid receptor antagonists (e.g., SR-141716, SR-147778), ghrelin antagonists, leptin, and β3 agonists. The amounts of these drugs administered may be chosen as appropriate with reference to the clinical doses thereof. The medicament of the present invention and the aforementioned drugs may be administered to the patient simultaneously or different times.

Another mode of embodiment of the present invention is a medicament consisting of a combination of two kinds or more of substances capable of binding to at least a portion of a cis-element of each of the mRNAs that encode two different kinds or more of target proteins to inhibit the binding of a cis-element binding factor to the cis-element.

Two different kinds or more of target proteins refer to, for example, two kinds or more of proteins selected from the group consisting of FGF21 and genes wherein the mRNA is instabilized by a cis-element binding factor as in FGF21, or wherein the translation from the mRNA is suppressed.

The above-described substance is specifically exemplified by substances capable of binding to at least a portion of a cis-element of the mRNA that encodes each target protein to inhibit the binding of a cis-element binding factor (preferably an AU-rich-element binding factor) to the cis-element (preferably an AU-rich element).

Here, examples of cis-elements and cis-element-binding factors include the aforementioned cis-elements and cis-element-binding factors.

In more detail, the substance is a nucleic acid analogue having a base sequence complementary to at least a portion of a cis-element of the mRNA that encodes each target protein and a base sequence complementary to a portion of the untranslated region on the 5' side and/or 3' side of the cis-element, and capable of inhibiting the binding of a cis-element binding factor to the cis-element by binding specifically to at least a portion of the cis-element on the basis of the sequence specificity possessed by the base sequence complementary to the untranslated region, and is preferably an oligonucleotide or a salt thereof.

The nucleotide molecule constituting the oligonucleotide may be a nucleotide molecule undergoing one of the aforementioned chemical modifications. The oligonucleotide preferably contains LNA or BNA, more preferably contains BNA. The oligonucleotide may be an S-oligo-modified oligonucleotide (an oligonucleotide resulting from replacement of phosphate residues among the various nucleotides constituting an oligonucleotide with chemically modified phosphate residues of phosphorothioate).

Examples of substances capable of binding to at least a portion of a cis-element of the mRNA that encodes one target protein to inhibit the binding of a cis-element binding factor to the cis-element include oligonucleotides, LNA-containing oligonucleotides, BNA-containing oligonucleotides, and other nucleic acid analogues, or salts thereof; examples of preferable substances include LNA-containing oligonucleotides or salts thereof and BNA-containing oligonucleotides or salts thereof.

Examples of substances capable of binding to at least a portion of a cis-element of the mRNA that encodes another target protein to inhibit the binding of a cis-element binding factor include oligonucleotides, LNA-containing oligonucleotides, BNA-containing oligonucleotides, and other nucleic acid analogues, or salts thereof; examples of preferable substances include LNA-containing oligonucleotides or salts thereof and BNA-containing oligonucleotides or salts thereof.

One kind or more of the substance may be low molecular compounds or salts thereof. Such a low molecular compound can be acquired by the same technique as the above for a low molecular compound that binds to a cis-element of the mRNA of FGF21. Salts of the low molecular compound are exemplified by the same salts as the above-described salts of low molecular compounds that bind to a cis-element of the mRNA of FGF21.

Another mode of embodiment of the present invention is a prophylactic/therapeutic agent for lifestyle-related disease comprising a substance that inhibits the function of ZFP36L2 or ZFP36L1.

Here, ZFP36L2 and ZFP36L1 are examples of "cis-element binding factors" as mentioned herein.

The substance that inhibits the function of ZFP36L2 or ZFP36L1 exhibits an effect equivalent to that of the substance of the present invention as a result of suppressing the expression of the protein, or binding to the protein, to inhibit the binding of a target mRNA such as FGF21 to a cis-element. Examples of such inhibitory substances include antisense nucleic acids, siRNAs, shRNAs, miRNAs or ribozyme nucleic acids against ZFP36L2 (SEQ ID NOs: 6, 8) or ZFP36L1 (SEQ ID NOs: 5, 7) mRNA, and neutralizing antibodies; aptamers or antagonist compounds against ZFP36L2 or ZFP36L1 protein. These inhibitory substances are readily available to those skilled in the art by applying, for example, a method described in WO2008/102777, on the basis of information on the base sequences and amino acid sequences of ZFP36L2 and ZFP36L1.

Specific examples of the lifestyle-related diseases are as described above.

The aforementioned substance may be directly used as a prophylactic or therapeutic agent, or may be used together with an additive similar to that in the medicament of the present invention.

In addition, the prophylactic or therapeutic agent can be used according to the administration method and dose mentioned above for the medicament of the present invention.

When base, amino acid and the like are indicated by abbreviations in the present specification, they are based on the abbreviations according to IUPAC-IUB Commission on Biochemical Nomenclature or conventional abbreviations in this field, and examples thereof are shown below. In addition, when an amino acid may have an optical isomer, it is an L form unless particularly indicated.

DNA: deoxyribonucleic acid
cDNA: complementary deoxyribonucleic acid
A: adenine
T: thymine
G: guanine
C: cytosine
RNA: ribonucleic acid
mRNA: messenger ribonucleic acid
dATP: deoxyadenosine triphosphate
dTTP: deoxythymidine triphosphate
dGTP: deoxyguanosine triphosphate
dCTP: deoxycytidine triphosphate
ATP: adenosine triphosphate
EDTA: ethylenediaminetetraacetic acid
SDS: dodecylsodium sulfate
Gly: glycine
Ala: alanine
Val: valine
Leu: leucine
Ile: isoleucine
Ser: serine
Thr: threonine
Cys: cysteine
Met: methionine
Glu: glutamic acid
Asp: aspartic acid
Lys: lysine
Arg: arginine
His: histidine
Phe: phenylalanine
Tyr: tyrosine
Trp: tryptophan
Pro: proline
Asn: asparagine
Gln: glutamine
pGlu: pyroglutamic acid
Sec: selenocysteine
THF: tetrahydrofuran
HPLC: high performance liquid chromatography
DMSO: dimethyl sulfoxide
PCR: polymerase chain reaction
RT-PCR: reverse transcription-polymerase chain reaction
FBS: fetal bovine serum
DMEM: Dulbecco's Modified Eagle Medium
MEM: Minimal Essential Medium

The SEQ ID NOs in the Sequence Listing in the present specification show the following sequences.
[SEQ ID NO: 1]
   shows the base sequence of human FGF21 cDNA (GenBank Accession No. NM_019113.2).
[SEQ ID NO: 2]
   shows the base sequence of oligonucleotide complementary to a portion of ARE-A.
[SEQ ID NO: 3]
   shows the base sequence of oligonucleotide complementary to a portion of ARE-B.
[SEQ ID NO: 4]
   shows the base sequence of oligonucleotide without complementarity to FGF21 mRNA.
[SEQ ID NO: 5]
   shows the base sequence of cDNA encoding human ZFP36L1 (GenBank Accession No. NM_004926.2).
[SEQ ID NO: 6]
   shows the base sequence of cDNA encoding human ZFP36L2 (GenBank Accession No. NM_006887.4).
[SEQ ID NO: 7]
   shows the base sequence of cDNA encoding mouse ZFP36L1 (GenBank Accession No. NM_007564.3).
[SEQ ID NO: 8]
   shows the base sequence of cDNA encoding mouse ZFP36L2 (GenBank Accession No. NM_001001806.2).
[SEQ ID NO: 9]
   shows the base sequence of cDNA encoding mouse FGF21 (GenBank Accession No. NM_020013.4).
[SEQ ID NO: 10]
   shows the base sequence of a primer for human β-actin mRNA amplification.
[SEQ ID NO: 11]
   shows the base sequence of a primer for human β-action mRNA amplification.
[SEQ ID NO: 12]
   shows the base sequence of a primer for human FGF21 mRNA amplification.
[SEQ ID NO: 13]
   shows the base sequence of a primer for human FGF21 mRNA amplification.
[SEQ ID NO: 14]
   shows the base sequence of a primer for mouse β-actin mRNA amplification.
[SEQ ID NO: 15]
   shows the base sequence of a primer for mouse β-actin mRNA amplification.
   (GenBank Accession No. NM_004926.2).
[SEQ ID NO: 16]
   shows the base sequence of a primer for mouse FGF21 mRNA amplification.
   (GenBank Accession No. NM_006887.4).
[SEQ ID NO: 17]
   shows the base sequence of a primer for mouse FGF21 mRNA amplification.
[SEQ ID NO: 18]
   shows the base sequence of oligonucleotide complementary to a portion of ARE-B.
[SEQ ID NO: 19]
   shows the base sequence of oligonucleotide complementary to a portion of ARE-B.
[SEQ ID NO: 20]
   shows the base sequence of oligonucleotide complementary to a portion of ARE-B.
[SEQ ID NO: 21]
   shows the base sequence of oligonucleotide complementary to a portion of ARE-B.
[SEQ ID NO: 22]
   shows the base sequence of oligonucleotide complementary to a portion of ARE-B.
[SEQ ID NO: 23]
   shows the base sequence of oligonucleotide complementary to a portion of ARE-B.
[SEQ ID NO: 24]
   shows the base sequence of oligonucleotide complementary to a portion of ARE-B.
[SEQ ID NO: 25]
   shows a partial base sequence at the 826th to 843rd from the 5' terminal side of human FGF21 mRNA.
[SEQ ID NO: 26]
   shows a partial base sequence at the 791st to 860th from the 5' terminal side of human FGF21 mRNA.
[SEQ ID NO: 27]
   shows the base sequence of a primer for human β-action mRNA amplification.
[SEQ ID NO: 28]
   shows the base sequence of a primer for human β-actin mRNA amplification.
[SEQ ID NO: 29]
   shows the base sequence of a probe for human β-actin.
[SEQ ID NO: 30]
   shows the base sequence of a primer for human FGF21 mRNA amplification.
[SEQ ID NO: 31]
   shows the base sequence of a primer for human FGF21 mRNA amplification.
[SEQ ID NO: 32]
   shows the base sequence of a probe for human FGF21.
[SEQ ID NO: 33]
   shows the base sequence of a primer for mouse β-actin mRNA amplification.
[SEQ ID NO: 34]
   shows the base sequence of a primer for mouse β-actin mRNA amplification.
[SEQ ID NO: 35]
   shows the base sequence of a probe for mouse β-actin.
[SEQ ID NO: 36]
   shows the base sequence of a primer for mouse FGF21 mRNA amplification.
[SEQ ID NO: 37]
   shows the base sequence of a primer for mouse FGF21 mRNA amplification.
[SEQ ID NO: 38]
   shows the base sequence of a probe for mouse FGF21.

The present invention is explained in more detail in the following by referring to Examples and Experimental Examples, which are not to be construed as limitative. Examples

### (Example 1) Synthesis of BNA^{NC}-adenosine monomer unit

A starting material 1 (680 mg, 1.17 mmol) and 6-N-benzoyladenine (1.4 g, 5.9 mmol) were suspended in toluene (11 mL). To the suspension was added N,O-bis-trimethylsilylacetamide (BSA), and the mixture was heated to 100°C and stirred for 1 hr. To the reaction mixture was added trimethylsilyl trifluoromethanesulfonate (TMSOTf), and the mixture was further stirred for 30 min. The reaction mixture was cooled and diluted with ethyl acetate (200 mL), and washed with saturated aqueous sodium hydrogen carbonate solution, water and saturated brine (each 100 mL). The obtained organic layer was dried over sodium sulfate and concentrated. The mixture was purified by silica gel column chromatography (hexane-ethyl acetate) to give the object protected BNA^{NC}-adenosine nucleoside (compound 2: 533 mg, 0.81 mmol).
¹H NMR (600MHz, CHLOROFORM-d) δ 0.91-1.20 (m, 28H), 2.68 (d,J=11.00, 1H),2.81 (s, 3H), 3.01(d,J=11.00, 1H), 3.73 (d,J=12.84, 1H), 4.04 (d,J=12.84, 1H), 4.50 (d,J=2.93, 1H), 4.77 (d,J=2.57, 1H), 6.79 (s, 1H), 7.52 (t,J=7.70, 2H), 7.61 (t,J=7.30, 1H), 8.03 (d,J=7.70, 2H), 8.35 (s, 1H), 8.81 (s, 1H), 9.17 (s, 1H) .

Compound 2 (530 mg, 0.81mmol) was dissolved in THF (10 mL), triethylamine (200 µL, 1.42 mmol) and triethylamine trihydrofluoride (460 µL, 2.84 mmol) were added, and the mixture was stirred at room temperature for 1.5 hr. The reaction mixture was concentrated, diluted with ethyl acetate (10 mL), and purified by silica gel column chromatography (ethyl acetate-methanol). (compound 3: 330 mg, 0.81 mmol)
¹H NMR (300MHz, DMSO-d6) 5 2.72 (s, 3H), 2.86 (s, 2H), 3.62 (m, 2H), 4.18 (dd,J=5.5, 3.2, 1H), 4.52 (d,J=3.0, 1H), 5.12 (t,J=5.9, 1H), 5.47 (d,J=5.5, 1H), 6.67 (s, 1H), 7.48-7.72 (m, 3H), 8.01-8.09 (m, 2H), 8.60 (s, 1H), 8.75 (s, 1H), 11.22 (br. s, 1H).

Compound 3 (300 mg, 0.72 mmol) was azeotropically dried with dry pyridine, and dissolved in pyridine (4 mL). 4,4'-Dimethoxytritylchloride (271 mg, 0.8 mmol) was added, and the mixture was stirred at room temperature for 12 hr. To the reaction mixture was added saturated aqueous sodium hydrogen carbonate solution (10 mL), and the aqueous layer was extracted with ethyl acetate (25 mL). The obtained organic layer was washed with saturated aqueous sodium hydrogen carbonate solution (10 mL). The organic layer was dried over anhydrous sodium sulfate, and concentrated. Purification by silica gel column chromatography (hexane-ethyl acetate) gave compound 4. (compound 4: 416 mg, 0.58 mmol)
¹H NMR (300MHz, CHLOROFORM-d) 5 2.78 (s, 3H), 2.82-3.11 (m, 3H), 3.38 (s, 2H), 3.77 (s, 6H), 4.39 (br. s, 1H), 4.67 (d,J=2.8, 1H), 6.75-6.89 (m, 5H), 7.15-7.64 (m, 12H), 8.00 (d, 2H), 8.33 (s, 1H), 8.79 (s, 1H), 9.17(br. s, 1H).

Compound 4 (410 mg, 0.57 mmol) was azeotropically dried twice with anhydrous acetonitrile, and dissolved in anhydrous acetonitrile (2.5 mL). N,N,N',N'-tetraisopropyl-2-cyanoethyl phosphordiamidite (200 µL, 0.63 mmol) and 4,5-dicyanoimidazole (71 mg, 0.6 mmol) were successively added. After stirring at room temperature for 2 hr, the reaction mixture was diluted with ethyl acetate (20 mL). Saturated aqueous sodium hydrogen carbonate solution (10 mL) was added to quench the reaction. The organic layer was further washed with saturated aqueous sodium hydrogen carbonate solution (10 mL), dried over anhydrous sodium sulfate and concentrated. Purification by silica gel column chromatography (Diol-silica gel (manufactured by Fuji Silysia Chemical LTD., hexane-acetone) gave the object BNA^{NC}-adenosine monomer. (compound 5: 480 mg, 0.52 mmol)
¹H NMR (300MHz, CHLOROFORM-d) δ 0.92-1.22 (m, 12H), 2.26-2.48 (m, 2H), 2.76, 2.78 (2s, 3H), 2.82-2.95 (m, 2H), 3.27-3.64 (m,4H), 3.78-3.79 (m, 6H), 4.44-4.59 (2m,1H), 4.81-4.89 (m, 1H), 6.75-6.89 (m, 5H), 7.16-7.66 (m, 12H), 7.99-8.07 (m, 2H), 8.35, 8.42 (2s, 1H), 8.84 (s, 1H), 9.05 (br. s, 1H).
³¹P NMR (121MHz) δ 149.08, 149.36

### (Example 2) Synthesis of BNA^{NC}-guanosine monomer unit

Starting material 1 (1.0 g, 1.7 mmol) and 6-O-diphenylcarbamoyl-2-N-acetylguanine (3.6 g, 8.6 mmol) were suspended in toluene (10 mL). To the suspension was added N,O-bis-trimethylsilylacetamide (BSA), and the mixture was stirred at 100°C for 30 min. To the reaction mixture was added trimethylsilyl trifluoromethanesulfonate (TMSOTf), and the mixture was further stirred for 30 min. The reaction mixture was cooled, diluted with ethyl acetate (50 mL), and washed with saturated aqueous sodium hydrogen carbonate solution, water and saturated brine (each 50 mL). The obtained organic layer was dried over sodium sulfate. The insoluble material was removed by filtration, and the filtrate was concentrated. The mixture was purified by silica gel column chromatography (hexane-ethyl acetate) to give the object protected BNA^{NC}-guanosine nucleoside (compound 6: 325 mg, 0.4 mmol).
¹H NMR (600MHz, CHLOROFORM-d) 5 ppm 0.77-1.19 (m, 28H), 2.60 (s, 3H), 2.67 (d,J=11.00Hz, 1H), 2.79 (s, 3H), 2.98 (d,J=11.00Hz, 1H), 3.72 (d,J=12.84Hz, 1H), 4.06 (d,J=12.84Hz, 1H), 4.22 (d,J=2.93Hz, 1H), 4.53 (d,J=2.93Hz, 1H), 6.63 (s, 1H), 7.12-7.56 (m, 10H), 8.02 (s, 1H), 8.29 (s, 1H).

Compound 6 (320 mg, 0.4 mmol) was dissolved in THF (5 ml), triethylamine (98 µL, 0.7 mmol) and triethylamine trihydrofluoride (228 µL, 1.4 mmol) were added, and the mixture was stirred at room temperature for 1.5 hr. The reaction mixture was concentrated, diluted with ethyl acetate (10 mL), and purified by silica gel column chromatography (ethyl acetate-methanol) to give compound 7. (compound 7: 180 mg, 0.32 mmol)
¹H NMR (300MHz, CHLOROFORM-d) δ 2.38 (s, 3H), 2.59-2.92 (m, 4H), 3.68 (s, 2H), 3.91 (br. s, 1H), 4.21 (br. s, 1H), 4.48 (d,J=2.64Hz, 1H), 4.58 (br. s, 1H), 6.57 (s, 1H), 7.11-7.54 (m, 10H), 8.25 (s, 1H), 8.92 (s, 1H).

Compound 7 (160 mg, 0.28 mmol) was azeotropically dried with dry pyridine, and dissolved in pyridine (1.5 mL). 4,4'-Dimethoxytritylchloride (108 mg, 0.32 mmol) was added, and the mixture was stirred at room temperature for 12 hr. To the reaction mixture was added saturated aqueous sodium hydrogen carbonate solution (10 mL), and the aqueous layer was extracted with ethyl acetate (20 mL). The obtained organic layer was washed with saturated aqueous sodium hydrogen carbonate solution (10 mL), dried over anhydrous sodium sulfate, and concentrated. Purification by silica gel column chromatography (hexane-ethyl acetate) gave compound 8. (compound 8: 176 mg, 0.20 mmol)
¹H NMR (300MHz, CHLOROFORM-d) 5 2.49 (s, 3H), 2.72 (d,J=8.50Hz, 1H), 2.78 (s, 3H), 2.85 (d,J=11.90Hz, 1H), 3.04 (d,J=11.90Hz, 1H), 3.35 (s, 2H), 3.75 (m, 6H), 4.47 (br, 1H), 4.70 (d,J=2.83Hz, 1H), 6.67 (s, 1H), 6.80 (d,J=8.69Hz, 4H), 7.14-7.52 (m, 19H), 8.15 (s, 1H), 8.22 (s, 1H).

Compound 8 (140 mg, 0.16 mmol) was azeotropically dried twice with anhydrous acetonitrile, and dissolved in anhydrous acetonitrile (1 mL). N,N,N',N'-tetraisopropyl-2-cyanoethyl phosphordiamidite (76 µL, 0.24 mmol) and 4,5-dicyanoimidazole (21 mg, 0.18 mmol) were successively added. After stirring at room temperature for 2 hr, N,N,N',N'-tetraisopropyl-2-cyanoethyl phosphordiamidite (38 µL, 0.12 mmol) and 4,5-dicyanoimidazole (10 mg, 0.09 mmol) were further added successively. After stirring at room temperature for 1 hr, the reaction mixture was diluted with ethyl acetate (10 mL), and saturated aqueous sodium hydrogen carbonate solution (5 mL) was added to quench the reaction. The organic layer was further washed with saturated aqueous sodium hydrogen carbonate solution (10 mL), dried over anhydrous sodium sulfate and concentrated. Purification by silica gel column chromatography (Diol-silica gel (manufactured by Fuji Silysia Chemical LTD., hexane-acetone) gave the object BNA^{NC}-guanosine monomer. (compound 9: 153 mg, 0.14 mmol)
¹H NMR (300MHz, CHLOROFORM-d) δ 0.92-1.19 (m, 12H), 2.27-2.49 (m, 2H), 2.58 (s, 3H), 2.74, 2.77 (2s, 3H), 2.82-3.06 (m, 2H), 3.27-3.73 (m, 4H), 3.77-3.78 (m, 6H), 4.20-4.45 (m, 1H), 4.73 (m, 1H), 6.68 (2s, 1H), 6.77-6.87 (m, 4H), 7.20-7.51 (m, 19H), 7.98 (s, 1H), 8.27, 8.31 (2s, 1H).
³¹P NMR (121MHz) TM148.72, 149.41
The BNA synthesis scheme of Examples 1 and 2 is shown in formula 2.

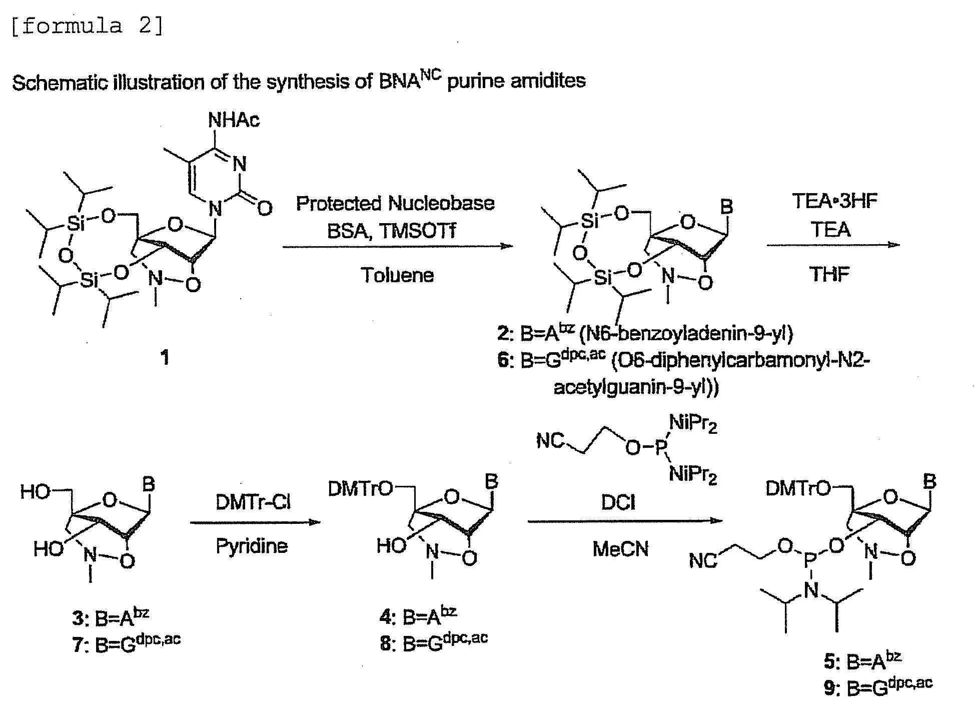

### (Example 3) Synthesis of BNA^{NC} oligonucleotide

Figure 1(D) shows the base sequence of an oligonucleotide complementary to a portion of ARE-B, which is one of AREs present in the 3'UTR of FGF21 mRNA (denoted as "α-F21-B (BNA)" or "TUP#001"). α-F21-B (BNA) consists of a base sequence complementary to a portion of ARE-B and a base sequence complementary to a portion of the 3'UTR continuous to the 5' side of ARE-B (see SEQ ID NO:3).
The BNA^{NC} oligonucleotide α-F21-B (BNA) (TUP#001), which consists of the base sequence shown by SEQ ID NO:3, was synthesized using an automated nucleic acid synthesizer (manufactured by Nihon Techno Service Co., MODEL:NTS H-8-SE) on the 0.2-micromol scale. The various reagents used for the synthesis were the commercial products: 10% dichloroacetic acid toluene solution, dry acetonitrile (Wako Pure Chemical), 0.35M benzylthiotetrazole acetonitrile solution, CapA, CapB, an oxidant (Glen Research). The phosphoroamidites of BNA^{NC} thymidine and 5-methylcytidine used were purchased from BNA Inc. The phosphoroamidites of BNA^{NC} adenosine and guanosine used were the compound 5 of Example 1 and the compound 9 of Example 2. The solid phase carrier used was Unysupport manufactured by Glen Research Corporation. A condensing reaction was carried out using 50 equivalents of amidite for a reaction time of 15 minutes to synthesize the title compound. The condensing reaction of the 1st base and 2nd base for the solid phase carrier was twice carried out, whereby the synthetic yield of the full-length oligonucleotide was improved.

The protected oligonucleotide analogue having the desired sequence was treated with 25% aqueous ammonia at 55°C to cleave out the oligomer from the solid phase carrier and deprotection was performed on the phosphorus atom and at the nucleic acid base site. After the solid phase carrier was filtered off, the remaining solvent was dried off. The residue was purified by ion exchange HPLC (AKTA explore 10s manufactured by GE Healthcare, column: SOURCE15Q 4.6/100PE, eluent A: 10 mM NaClO₄ 1 mM Tris, eluent B: 1M NaClO₄ 1 mM Tris gradient: B=0%-100% (17 min linear gradient), room temperature, flow rate 1.5 mL/min), and a peak having a dimethoxytrityl group at the 5'-end of the synthetic oligonucleotide was gathered. The desired product fraction was gathered and concentrated; 80% aqueous acetic acid solution (300 µL) was added to the residue, and this was allowed to stand for 20 minutes to thereby remove the dimethoxytrityl group. Water (300 µL) and 3M sodium acetate aqueous solution (120 µL) were added to the reaction mixture, and this was concentrated. After the concentration, the residue was filled up to 1 mL with water, after which desalination was twice performed using the NAP-10 column manufactured by GE Healthcare Co. to yield the desired oligonucleotide. The compound obtained was analyzed by ion exchange HPLC (AKTA explore 10s manufactured by GE Healthcare, column: SOURCE15Q 4.6/100 PE, eluent A: 10 mM NaClO₄ 1 mM Tris, eluent B: 1M NaClO₄ 1 mM Tris gradient: B=0%-100% (17 min linear gradient), room temperature, flow rate 1.5 mL/min) and was eluted at a retention time of 6.5 minutes. The mass of the compound obtained was identified by MALDI-TOF mass analysis (Calculated: 4379.1(M-1), Found: 4379.20).

### (Experimental Example 1) FGF21 expression control experiment

The amount of gene expressed was controlled with FGF21 as the target gene, using an oligonucleotide according to the present invention.

### (1-1) Designing of polynucleotide analogue

With reference to the base sequence of FGF21 mRNA registered with the NCBI database, two AREs present in the 3'UTR of the mRNA were identified. These two AREs (denoted as "ARE-A" and "ARE-B") were identified as the consensus sequence "UAUUUAUU" conserved among the biological species of human, rhesus macaque, mouse, rat, dog, horse and the like.
ARE-A corresponds to the region of the 814th to 821st bases from the 5' end of the human FGF21 mRNA shown by SEQ ID NO:1 (GenBank Accession No. NM_019113). ARE-B corresponds to the region of the 835th to 842nd bases from the 5' end.
Oligonucleotides complementary to portions of ARE-A and ARE-B were designed. Figure 1 shows partial base sequences of the FGF21 mRNA 3'-UTR around ARE-A and ARE-B and the base sequences of the oligonucleotides designed.
Figure 1(A) shows the base sequence of a portion containing ARE-A and ARE-B (the 791st to 860th bases from the 5' side end) (SEQ ID NO:26) in the full-length 940 bases of FGF21 mRNA (see SEQ ID NO:1). The underlined portion in the base sequence indicates the "AUUUA pentamer".
Figure 1(B) shows the base sequence of an oligonucleotide (denoted as "α-F21-A (LNA)") complementary to a portion of ARE-A. α-F21-A (LNA) consists of a base sequence complementary to a portion of ARE-A (cARE) and a base sequence complementary to a portion of the 3'UTR continuous to the 3' side of ARE-A (cUTR) (see SEQ ID NO:2). The length of α-F21-A (LNA) is 12mer.
Figure 1(C) shows the base sequence of an oligonucleotide (denoted as "α-F21-B (LNA)" or (TUP#002)) complementary to a portion of ARE-B. α-F21-B (LNA) (TUP#002) consists of a base sequence complementary to a portion of ARE-B and a base sequence complementary to a portion of the 3'UTR continuous to the 5' side of ARE-B (see SEQ ID NO: 3).
The length of α-F21-B (LNA) is 12mer. α-F21-A (LNA) and α-F21-B (LNA) are synthetic oligonucleotides consisting of LNAs. α-F21-A (LNA) and α-F21-B (LNA) were obtained by consignment synthesis (Gene Design Inc.).
Syntheses of α-F21-A (LNA) and α-F21-B (LNA) were performed in conformity to a method known per se. That is, they were synthesized by performing deprotection using ammonia after solid phase synthesis was performed by the ordinary phosphoroamidite method (e.g., method described in Tetrahedron Letters, vol.22 (1981) 1859-1862, and Chemical Reviews, vol.90 (1990) 543-584). The oligonucleotides obtained were purified using reversed-phase HPLC or ion exchange HPLC. After the purification, the oligonucleotides obtained were subjected to reversed-phase HPLC to determine the purity, and applied to a MALDI-TOF mass analyzer to determine the molecular weight.

The molecular weight of α-F21-B (LNA) was 4029.6 (calculated value 4031.7(M+1)).

### (1-2) Introduction of oligonucleotide

Human hepatocyte HepG2 cells were seeded to a 12-well plate at 2.0×10⁵ cells/well, and cultured using a DMEM medium containing 10% FBS. After cultivation for 24 hours, an oligonucleotide (α-F21-B (LNA) or control oligonucleotide) was introduced into the cells by lipofection (DharmaFECT 4, Thermo Fisher Scientific). For control, an oligonucleotide consisting of LNAs of a base sequence (5'-AGATGAATAAA-3'; SEQ ID NO: 4) lacking complementarity to FGF21 mRNA was used.
40 pmol of the oligonucleotide was used after being diluted with 50 µL of Opti-MEM (Life Technologies Corporation). 1.6 µL of Dharma FECT 4 was diluted with 50 µL of Opti-MEM. Subsequently, the dilution was allowed to stand at room temperature for 5 minutes, the oligonucleotide dilution and the DharmaFECT 4 dilution were mixed, and the mixture was allowed to stand for 20 minutes, after which the entire volume was added to each well of a 12-well plate.

### (1-3) Evaluation of the amount of FGF21 expressed

24 hours after introduction of the oligonucleotide, the cells were recovered, and mRNA extraction was performed as described below; FGF21 mRNA was quantified by a real time PCR method using the extracted mRNA.
For 1 µg of the purified Total RNA, cDNA was synthesized using High Capacity cDNA Reverse Transcription Kit with RNase Inhibitor (Applied biosystems), which was diluted to a total of 100 µl using Milli-Q; using the cDNA at 1 µl/reaction (15 µl) for β-actin and at 3 µl/reaction (15 µl) for FGF21, quantitation was performed with n=3 or more using Fast SYBRR Green Master Mix (Applied biosystems), Applied Biosystems StepOnePlus. Quantitative values were calculated with the mean ratio of the amount of FGF21 mRNA/the amount of β-actin mRNA in the control as 1. Values other than those above were determined according to the protocols of Applied biosystems Co. (see below). The sequences of the primers used are shown below. High Capacity cDNA Reverse Transcription Kit with RNase Inhibitor protocol:
http://www3.appliedbiosystems.com/cms/groups/mcb_support/docum ents/generaldocuments/cms_042557.pdf
Fast SYBRR Green Master Mix protocol:
http://www.appliedbiosystems.co.jp/website/PDFOUT/127225

primer for human β-actin:
   Forward: 5'-TGGATCAGCAAGCAGGAGTATG-3' (SEQ ID NO: 10)
   Reverse: 5'-GCATTTGCGGTGGACGAT-3' (SEQ ID NO: 11)
primer for human FGF21:
   Forward: 5'-TGAAGCCGGGAGTTATTCAAA-3' (SEQ ID NO: 12)
   Reverse: 5'-GCCGCTGGCACAGGAA-3' (SEQ ID NO: 13)
primer for mouse β-action:
   Forward: 5'-CCAGTTCGCCATGGATGAC-3' (SEQ ID NO: 14)
   Reverse: 5'-ATGCCGGAGCCGTTGTC-3' (SEQ ID NO: 15)
primer for mouse FGF21:
   Forward: 5'-GTACCTCTACACAGATGACGACCAA-3' (SEQ ID NO: 16)
   Reverse: 5'-CGCCTACCACTGTTCCATCCT-3' (SEQ ID NO: 17)

### (1-4) Results

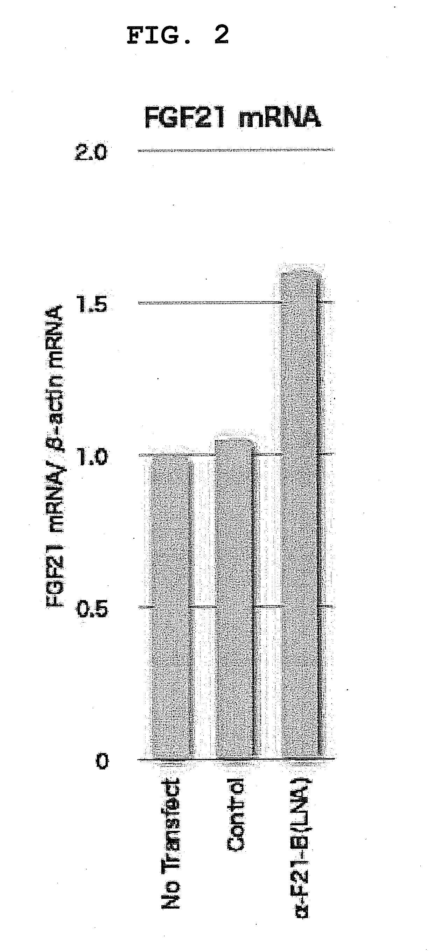

The results of the real time PCR for human hepatocyte HepG2 cells are shown in Figure 2. The amount of FGF21 mRNA is expressed as a value standardized with the amount of β-actin mRNA. An increase in the amount of FGF21 mRNA was confirmed in the cells transfected with α-F21-B (LNA), compared with the cells not transfected with the oligonucleotide and the cells transfected with the control oligonucleotide.

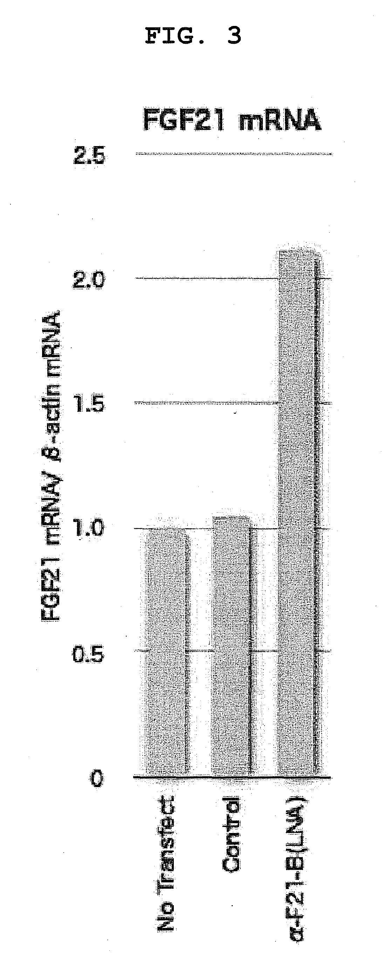

Figure 3 shows the results of a similar experiment conducted using mouse hepatocyte H2.35 cells. As with human cells (HepG2), an increase in the amount of FGF21 mRNA was confirmed in the cells transfected with α-F21-B (LNA), compared with the cells not transfected with the oligonucleotide and the cells transfected with the control oligonucleotide.
These results showed that using α-F21-B (LNA), the amount expressed of FGF21 which is the target gene could be enhanced specifically in the hepatocytes of human or mouse derivation.

### (Experimental Example 2) Changes in the expression of FGF21 by BNA^{NC} oligonucleotide

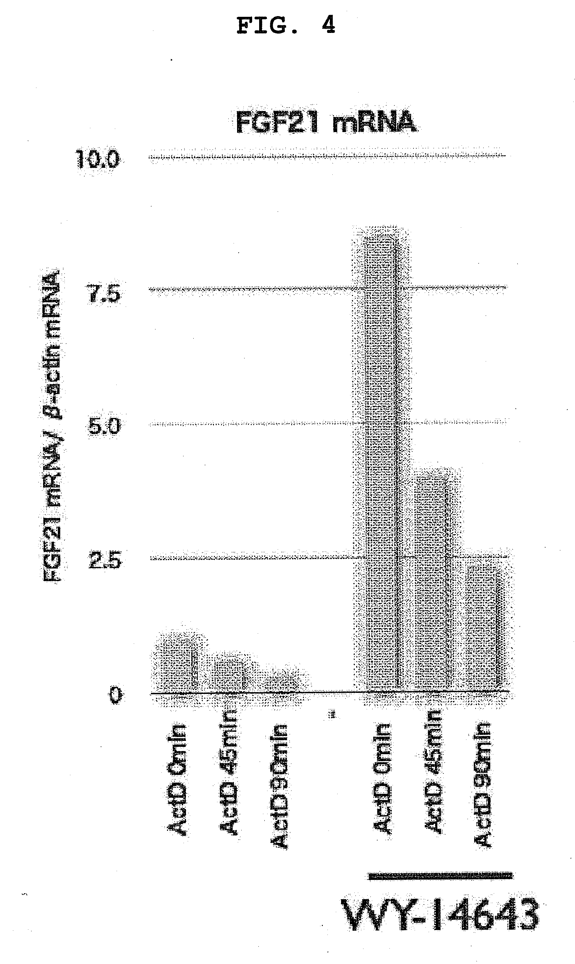

The oligonucleotide α-F21-B (BNA) obtained in Example 3 has the base sequence shown by SEQ ID NO:3, wherein all constituent monomers have been replaced by the BNA^{NC} monomer. With this in mind, α-F21-B (LNA), which has the base sequence shown by SEQ ID NO:3, wherein all constituent monomers have been replaced by the LNA monomer, was evaluated in the same manner as Experimental Example 1 to determine the effects of the oligonucleotide on the amount of FGF21 mRNA in the cultured hepatocyte line.
Of the culture cells used, the human hepatocyte line HepG2 had α-F21-B(BNA) introduced thereinto (final concentration 40 nM, α-F21-B (LNA) as control substance) in conformity to the method of Experimental Example 1; 24 hours after the introduction, total mRNA was extracted from the cells, and the FGF21 mRNA contained was quantified by a conventional method of real time PCR.
For the mouse hepatocyte strain H2.35, the method of Experimental Example 1 was modified as described below. That is, since it is known that Peroxisome proliferator-activated receptor (PPAR)-α agonist induces accentuation of the expression of FGF21 mRNA in hepatocytes in the publicly known literature (Cell Metab 2007 5(6): 415-425, Cell Metab 2007 5(6): 426-437, Biochem Biophys Res Commun 2007 360(2): 437-440), a PPAR-α agonist, WY-14643 (manufactured by Sigma) (200 µM), was allowed to co-present in the medium simultaneously with the introduction of an oligonucleotide, and the effects of the oligonucleotide were examined while in a state where the amount of FGF21 mRNA transcribed in the cells were amplified. WY-14643 was dissolved in DMSO, and used at a final concentration of 200 µM. As shown in Figure 4, it was confirmed that the amount of FGF21 mRNA in H2.35 cells is increased by WY-14643, and that the amount of the mRNA decreases under transcription-blocked conditions in the presence of actinomycin D (manufactured by Calbiochem) (5 µg/ml, three groups of 0 min, 45 min and 90 min), which is an RNA synthesis inhibitor. Actinomycin D was dissolved in DMSO and used at a final concentration of 5 µg/ml. This finding strongly suggested that FGF21 mRNA amplified by the PPAR-α agonist occurred unstably under the control of transcription and decomposition in the cells.

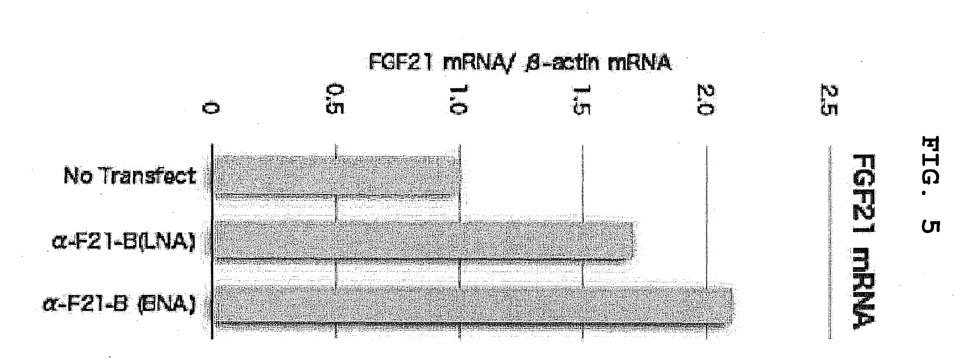

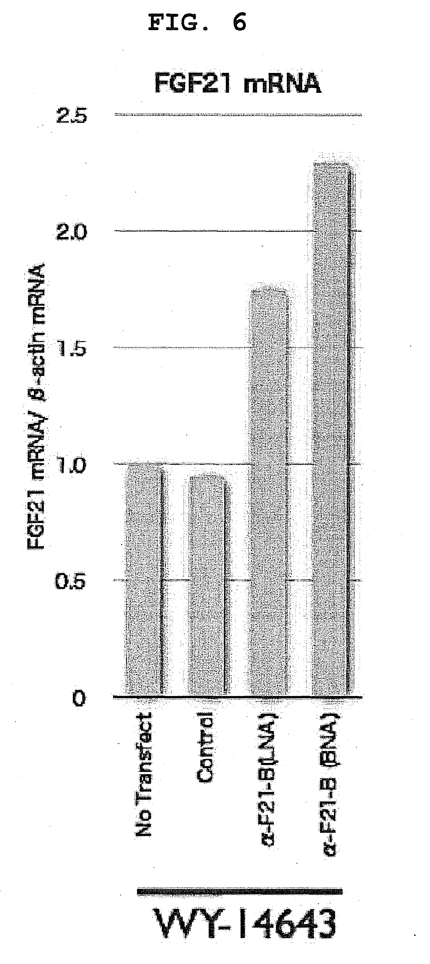

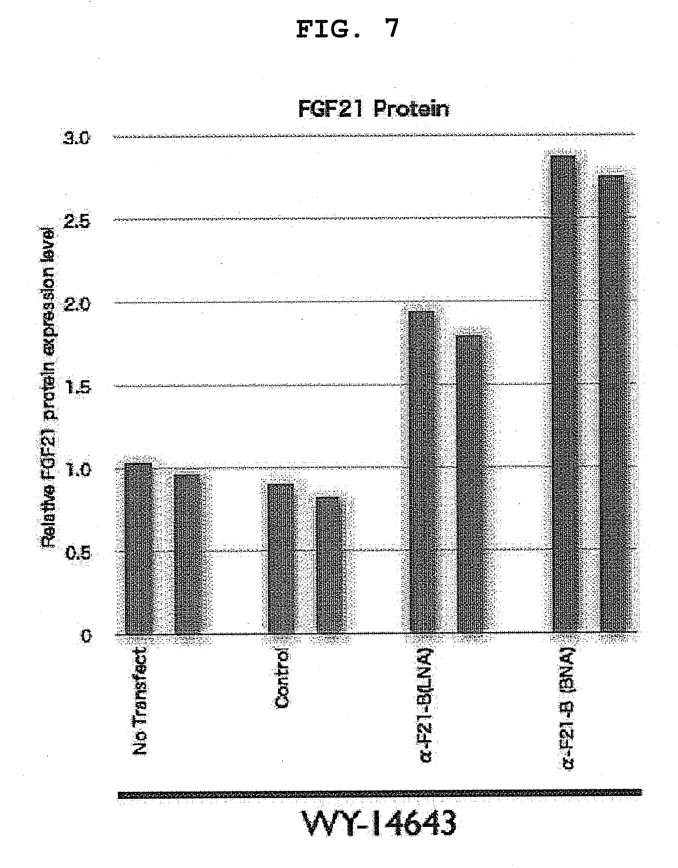

Shown in Figure 5 and Figure 6 are the results of an examination of the effects of introduction of an oligonucleotide on the amount of FGF21 mRNA in each of HepG2 (in the absence of WY-14643) and H2.35 (in the presence of WY-14643), respectively. Whether in human (HepG2) or in mouse (H2.35) cells, α-F21-B (BNA) increased the amount of each FGF21 mRNA; this effect was more potent than that of the same concentration of α-F21-B (LNA). For H2.35, the culture supernatant was recovered 48 hours after introduction of the oligonucleotide, and centrifuged at 1000xg for 10 minutes, after which the supernatant was recovered, using 50 µl (/well) of which quantitation of FGF21 protein was performed using a Human FGF-21 ELISA Kit (Millipore EZHFGF21-19K) by the ELISA method. The operation was performed according to the protocol attached to the kit. As a result, an increase in the amount of FGF21 protein was noted in the culture supernatants of the cells transfected with α-F21-B (BNA) or α-F21-B (LNA), the amount increased being larger with α-F21-B (BNA) than with α-F21-B (LNA) (Figure 7). The LNA oligonucleotide shown by SEQ ID NO:4, which is non-complementary to FGF21 mRNA and was used as the comparator in Experimental Example 1 as well (Control) did not influence the amount of FGF21 mRNA or the amount of protein in H2.35.

It was demonstrated from the results above that the BNA^{NC} oligonucleotide α-F21-B (BNA), like α-F21-B (LNA), accentuated the expression of FGF21 in both humans and mice, α-F21-B (BNA) being dominant in terms of the activity.

### (Experimental Example 3) Identification of ARE-binding factor

The results of Experimental Examples 1 and 2 suggested that α-F21-B (LNA) and α-F21-B (BNA) bind to ARE-B of the FGF21 gene to inhibit the binding of ARE-binding factor to ARE-B, and inhibit the function of negative control of expression of ARE-binding factor, thereby selectively enhancing the amount of FGF21 mRNA expressed. Hence, next, an attempt was made to identify an ARE-binding factor whose binding to ARE-B is inhibited by α-F21-B (LNA) or α-F21-B (BNA), and analyze the function thereof. First, for the purpose of identifying an ARE-binding factor that binds to ARE-B, by immunoprecipitation with FGF21 mRNA as bait (decoy) and a proteome analysis using a mass spectrometer, analysis was performed for a cis-element binding protein that binds to ARE-B of FGF21 mRNA.

### (3-1) Synthesis of FGF21 mRNA bait

FGF21 mRNA was synthesized by in vitro translation. The base sequence of the 778-876th bases from the 5' side end of FGF21 mRNA (SEQ ID NO:1) was amplified by PCR using a primer having a T7 promoter sequence at the 5' end, and RNA was synthesized using the MEGAscript T7 kit (Ambion) per the protocol attached. A reaction was carried out to covalently bind Flag-hydrazide to the 3' end of the synthesized FGF21 mRNA to label the 3' end of FGF21 mRNA with Flag according to a publicly known method (see "Programmable ribozymes for mischarging tRNA with nonnatural amino acids and their applications to translation." Methods, 2005, Vol,36, No.3, p.239-244). The labeled mRNA was purified using the RNeasy Mini Kit of Qiagen Co.

### (3-2) Immunoprecipitation and protein identification

10 pmol of the purified Flag-labeled FGF21 mRNA was mixed with anti-Flag antibody beads (Sigma), and the reaction was carried out at 4°C for 1 hour. Thereafter, 3 mg of cell extract protein extracted from 293T cells cultured using a DMEM medium containing 10% FBS was added, and a further reaction was carried out at 4°C for 1 hour. After the non-bound protein was washed down, RNA and RNA-binding protein were eluted with a Flag peptide. The sample obtained by the elution was treated with lysyl endopeptidase, and analyzed using the LC-MS/MS technique (see "A direct nanoflow liquid chromatography-tandem mass spectrometry system for interaction proteomics." Analytical Chemistry, 2002, Vol.74, No.18, p.4725-4733), which is a publicly known method. The mass spectrometer used was QSTAR XL (Applied Biosystems).

### (3-3) Results

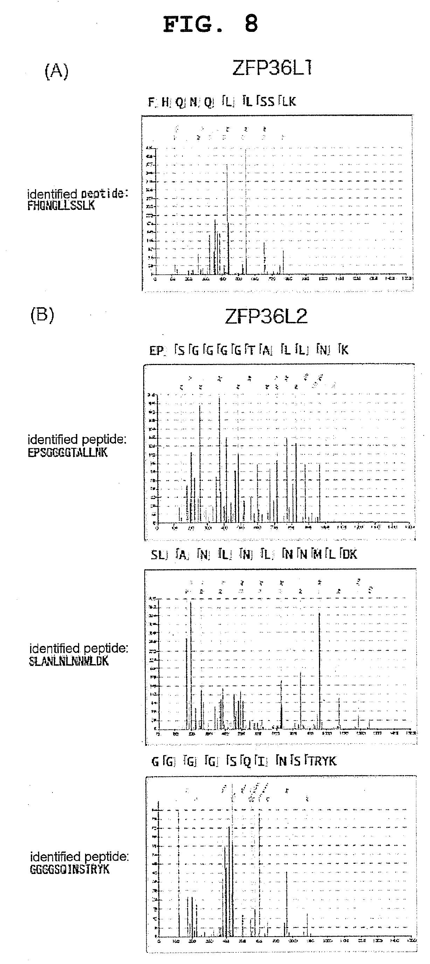

LC-MS/MS identified "ZFP36L1" and "ZFP36L2" as proteins that bind to the 3' end region of FGF21 mRNA (MS spectral data are shown in Figure 8 (1 peptide for ZFP36L1, 3 peptides for ZFP36L2). ZFP36L1 and ZFP36L2, along with ZFP36 (synonymized by TTP), form a family of ARE-binding factors (hereinafter referred to as "ZFP36 family"). The ZFP36 family has been reported to bind to AREs to instabilize mRNA in functioning to promote the decomposition (see "Tristetraprolin and its family members can promote the cell-free deadenylation of AU-rich element-containing mRNAs by poly(A) ribonuclease." Molecular Cell Biology, 2003, Vol.23, No.11, p.3798-812).
These results strongly suggested that the ARE-binding factors whose binding to ARE-B is inhibited by α-F21-B (LNA) and α-F21-B (BNA) might be ZFP36L1 and ZFP36L2.

### (Experimental Example 4) Evaluation of capability of ZFP36L1 and ZFP36L2 for binding to FGF21 mRNA and capability of α-F21-B (LNA) for inhibition of the binding

An investigation was performed to determine whether ZFP36L1 and ZFP36L2 were capable of binding to ARE-B of FGF21 mRNA, and whether α-F21-B (LNA) was capable of inhibiting the binding of the ZFP family to ARE-B.
According to the method explained in the "(3-1) Synthesis of FGF21 mRNA bait" in Experimental Example 3, 3'UTR of FGF21 mRNA comprising ARE-A and -B was synthesized by in vitro translation, and labeled with a Flag peptide. The 3'UTR used was the region of the 778-876th bases from the 5' side end of FGF21 mRNA (see SEQ ID NO:1).
According to the method explained in the "(3-2) Immunoprecipitation" in Experimental Example 3, cell extract protein extracted from 293T cells (5 mg/500 µl) was added to 10 pmol of purified Flag-labeled human FGF21 3'UTR-RNA (hereinafter referred to as "FGF21 3'UTR-Flag"); α-F21-A (LNA), α-F21-B (LNA) or the above-described control oligonucleotide was further added to obtain a final concentration of 100 µM; the mixture was immunoprecipitated using Flag M2 antibody beads, then eluted using the Flag peptide; the resulting sample was subjected to Western blotting using an anti-ZFP36L1 antibody (Cell signaling).

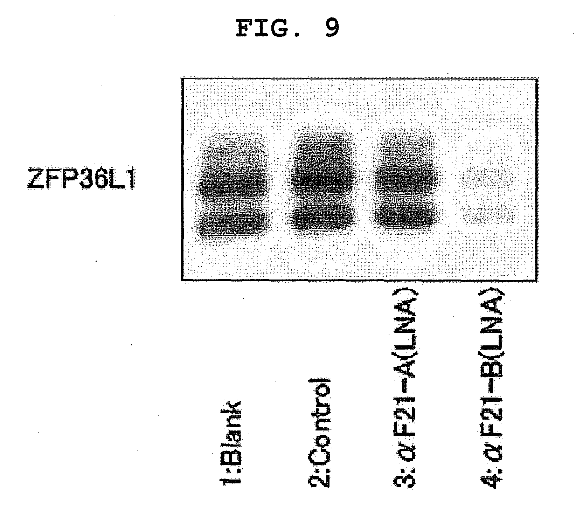

The results of the Western blotting are shown in Figure 9. In the sample obtained by mixing the cell extract protein with the FGF21 3'UTR-Flag and immunoprecipitating the same with the anti-ZFP36L1-antibody (lane 1), ZFP36L1 was detected. This shows that ZFP36L1 is capable of binding to FGF21 3'UTR-Flag, and also shows that ZFP36L1 possesses the capability of binding to the 3'UTR of FGF21. Similar results were obtained for ZFP36L2 (the same applies below with regard to Experimental Example 4).
On lane 4, where cell extract protein and FGF21 3'UTR-Flag were reacted in the presence of α-F21-B (LNA) containing a base sequence complementary to ARE-B of FGF21 mRNA, the detection signal for ZFP36L1 decreased remarkably. Meanwhile, on lanes 2 and 3, where the reaction was carried out with the addition of α-F21-A (LNA) and control oligonucleotide, no reduction was noted in the detection signal for ZFP36L1.
These results demonstrated that ZFP36L1 and ZFP36L2 bound to the ARE-B of FGF21 mRNA 3'UTR, and that α-F21-B (LNA) is capable of remarkably inhibiting the binding of ZFP36L1 and ZFP36L2 to ARE-B.

### (Experimental Example 5) Functional analysis of ZFP36L1 and ZFP36L2

To clarify the functions of ZFP36L1 and ZFP36L2 in controlling the expression of FGF21, changes in the amount of FGF21 expressed when suppressing the expression of ZFP36L1 and ZFP36L2 using RNAi were examined.
The cells were seeded to a 12-well plate at 2.0×10⁵ cells/well, and cultured using a DMEM medium containing 10% FBS. After cultivation for 24 hours, siRNA was transfected to the cells by lipofection (DharmaFECT 2, Thermo Scientific). 16 pmol of each oligonucleotide was used after being diluted with 50 µL of Opti-MEM (Life Technologies Corporation). Also, 1.6 µL of DharmaFECT 2 was diluted with 50 µL of Opti-MEM. Subsequently, the dilution was allowed to stand at room temperature for 5 minutes, the oligonucleotide dilution and the DharmaFECT 2 dilution were mixed, and the mixture was allowed to stand for 20 minutes, after which the entire volume was added to each well of a 12-well plate.
The siRNAs used were purchased from Invitrogen Co. The siRNA against ZFP36L1 and ZFP36L2 used were ZFP36L1/L2 RNAi #1 (Cat. No. HSS101104, HSS101101) and ZFP36L1/L2 RNAi #2 (Cat. No. HSS101105, HSS101102). The control siRNA (Cont. RNAi #1, #2) used was Stealth RNAi Negative Control (Cat. No. 12935-100) .
48 hours after introduction of the oligonucleotide, the cells were recovered, and real time PCR was performed using the mRNA extracted to evaluate the amount of FGF21 expressed.

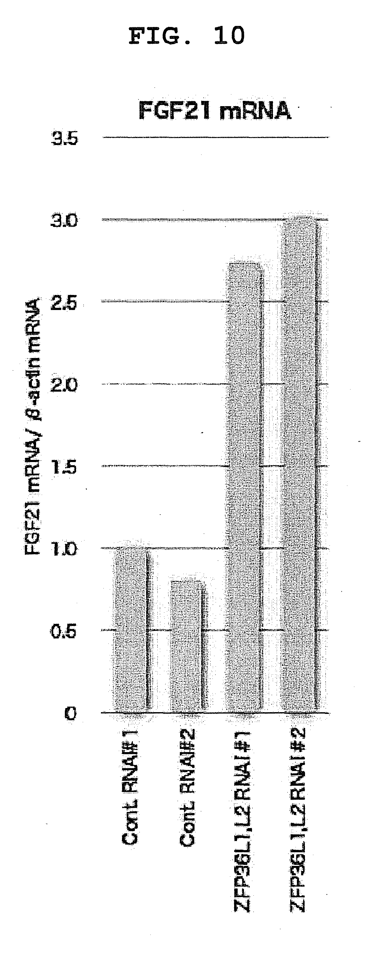

The results of the real time PCR are shown in Figure 10. The amount of FGF21 mRNA is expressed as a value standardized with the amount of β-actin mRNA. An increase in the amount of FGF21 mRNA was confirmed in the cells transfected with ZFP36L1/L2 RNAi #1 or #2, compared with the cells transfected with Cont.RNAi #1 or #2.
The above-described finding that the amount of FGF21 expressed was remarkably enhanced by introducing an siRNA that inhibits the expression of ZFP36L1 and ZFP36L2 shows that ZFP36L1 and ZFP36L2 functions in instabilizing FGF21 mRNA and promoting the decomposition to negatively control the expression of FGF21.

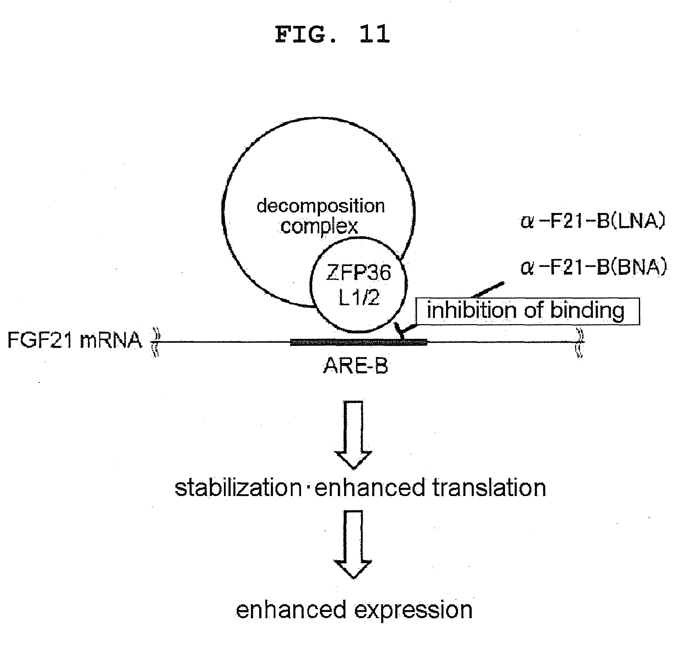

The above results of Experimental Examples 1 to 5 showed that α-F21-B (BNA) (TUP#001) and α-F21-B (LNA) (TUP#002) bound to ARE-B of FGF21 mRNA to inhibit the binding of ZFP36L1 and ZFP36L2 to ARE-B, and selectively enhanced the expression of FGF21 mRNA by suppressing the function of negatively controlling the expression of ZFP36L1 and ZFP36L2 to stabilize FGF21 mRNA and promote the translation, by this binding inhibition (see Figure 11).

### (Example 4) Design and synthesis of oligonucleotide complementary to a sequence in the vicinity of ARE-B of FGF21 mRNA

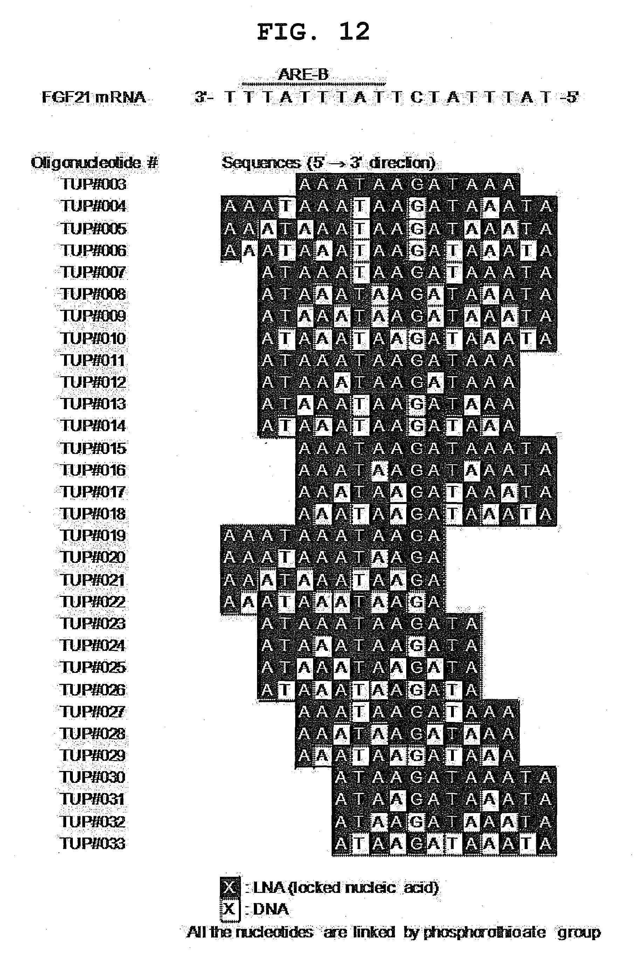

With the results of Experimental Examples 1 to 4 using α-F21-B (BNA) (TUP#001) and α-F21-B (LNA) (TUP#002) in mind, oligonucleotides complementary to a sequence in the vicinity of a target sequence to which they bind were designed (TUP#003 - TUP#033). Specifically, base sequences were selected to meet the criteria:
(1)
   (i) being a sequence in the vicinity of a cis-element (ARE-B) that binds to TUP#001 and TUP#002, and
   (ii) being a base sequence conserved among the four species of human, rhesus macaque, rat, and mouse; and
(2) TUP#003 to TUP#033 were designed as oligonucleotides complementary to the base sequences selected in (1) above.
The relationships between TUP#003 to TUP#033 and FGF21 mRNA are shown in Figure 12. These oligonucleotides are 12mer to 18mer; all or some of the monomers are LNA, and all phosphate-binding sites have a phosphorothioate modification. The base sequences of TUP#001 to TUP#033 are shown in Table 1.

**Table 1**

| TUP# | Base sequence¹⁾ | Sequence ID number | Chemical modification of phosphate residue among various nucleosides that constitute oligonucleotide |
|---|---|---|---|
| #001 α-F21-B(BNA) | A'A'A'T'A'A'G'A'T'A'A'A' | 3 | None |
| #002 α-F21-B(LNA) | AAATAAGATAAA | 3 | None |
| #003 | AAATAAGATAAA | 3 | Phosphorothioate |
| #004 | AAAtAAAtAAgATAaATA | 18 | PhosDhorothioate |
| #005 | AAaTaAAtAAgATaAaTA | 18 | PhosDhorothioate |
| #006 | AaAtAaAtAAgAtAaAtA | 18 | PhosDhorothioate |
| #007 | ATAAAtAAGAtAAATA | 19 | PhosDhorothioate |
| #008 | ATAaATaAGaTAaATA | 19 | PhosDhorothioate |
| #009 | ATaAaTaAGaTaAaTA | 19 | PhosDhorothioate |
| #010 | AtAaAtAagAtAaAtA | 19 | PhosDhorothioate |
| #011 | ATAAATAAGATAAA | 20 | PhosDhorothioate |
| #012 | ATAAaTAAGaTAAA | 20 | PhosDhorothioate |
| #013 | ATaAAtAAgATaAA | 20 | PhosDhorothioate |
| #014 | AtAaAtAAgAtAaA | 20 | PhosDhorothioate |
| #015 | AAATAAGATAAATA | 21 | PhosDhorothioate |
| #016 | AAATaAGATaAATA | 21 | PhosDhorothioate |
| #017 | AAaTAaGAtAAaTA | 21 | PhosDhorothioate |
| #018 | AaAtAaGAtAaAtA | 21 | PhosDhorothioate |
| #019 | AAATAAATAAGA | 22 | PhosDhorothioate |
| #020 | AAAtAAATaAGA | 22 | PhosDhorothioate |
| #021 | AAaTaAAtAaGA | 22 | PhosDhorothioate |
| #022 | AaAtAaaTaAgA | 22 | PhosDhorothioate |
| #023 | ATAAATAAGATA | 23 | PhosDhorothioate |
| #024 | ATAaATAAgATA | 23 | PhosDhorothioate |
| #025 | ATaAaTAaGaTA | 23 | PhosDhorothioate |
| #026 | AtAaAtaAgAtA | 23 | PhosDhorothioate |
| #027 | AAAtAAGAtAAA | 3 | PhosDhorothioate |
| #028 | AAaTaAGaTaAA | 3 | PhosDhorothioate |
| #029 | AaAtAagAtAaA | 3 | PhosDhorothioate |
| #030 | ATAAGATAAATA | 24 | PhosDhorothioate |
| #031 | ATAaGATAaATA | 24 | PhosDhorothioate |
| #032 | ATaAgATaAaTA | 24 | PhosDhorothioate |
| #033 | AtAaGatAaAtA | 24 | PhosDhorothioate |

1) The abbreviations used for base sequences in Table 1 mean the following.
a, t, g: native deoxyribonucleotide
A, T, G: LNA modified ribonucleotide
A', T', G': BNA^{NC} modified ribonucleotide The LNA modified ribonucleotide represented by A, T, G in Table 1 means ribonucleotide represented by the formula 3.

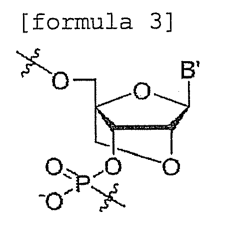

wherein B' is a nucleic acid base.

In addition, the BNA^{NC} modified ribonucleotide represented by A', T', G' in Table 1 means ribonucleotide represented by the formula 4.

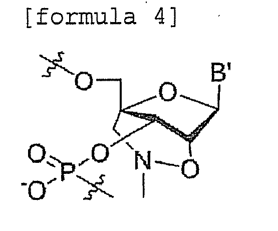

wherein B' is as defined above.

Synthesis of the oligonucleotides TUP#003 - #033 designed as mentioned above was entrusted to GeneDesign Inc. (Osaka).

TUP#003 - #033 were synthesized according to the method used for the synthesis of the aforementioned α-F21-A (LNA) and α-F21-B (LNA). The mass measurement results of TUP#003 - #033 are shown in Table 2.

**Table 2**

| No. | Mw: calculated | Found |
|---|---|---|
| TUP#003 | 4206.4 (M-1) | 4204.6 |
| TUP#004 | 6220.1 (M-1) | 6220.2 |
| TUP#005 | 6164.1 (M-1) | 6165.8 |
| TUP#006 | 6108.1 (M-1) | 6110.6 |
| TUP#007 | 5561.5 (M-1) | 5562.7 |
| TUP#008 | 5505.5 (M-1) | 5508.3 |
| TUP#009 | 5449.5 (M-1) | 5449.4 |
| TUP#010 | 5393.5 (M-1) | 5395.9 |
| TUP#011 | 4912.0 (M-1) | 4914.6 |
| TUP#012 | 4856.0 (M-1) | 4858.7 |
| TUP#013 | 4800.0 (M-1) | 4801.9 |
| TUP#014 | 4743.9 (M-1) | 4745.8 |
| TUP#015 | 4912.0 (M-1) | 4914.9 |
| TUP#016 | 4856.0 (M-1) | 4858.3 |
| TUP#017 | 4800.0 (M-1) | 4800.5 |
| TUP#018 | 4743.9 (M-1) | 4746.8 |
| TUP#019 | 4206.4 (M-1) | 4207.5 |
| TUP#020 | 4150.4 (M-1) | 4152.1 |
| TUP#021 | 4094.4 (M-1) | 4096.8 |
| TUP#022 | 4038.4 (M-1) | 4041.0 |
| TUP#023 | 4197.4 (M-1) | 4199.4 |
| TUP#024 | 4141.4 (M-1) | 4142.1 |
| TUP#025 | 4085.4 (M-1) | 4085.9 |
| TUP#026 | 4029.4 (M-1) | 4030.9 |
| TUP#027 | 4150.4 (M-1) | 4151.0 |
| TUP#028 | 4094.4 (M-1) | 4096.2 |
| TUP#029 | 4038.4 (M-1) | 4040.2 |
| TUP#030 | 4197.4 (M-1) | 4198.1 |
| TUP#031 | 4141.4 (M-1) | 4142.3 |
| TUP#032 | 4085.4 (M-1) | 4087.0 |
| TUP#033 | 4029.4 (M-1) | 4030.8 |

### (Experimental Example 6) Evaluation of FGF21 mRNA expression elevation activity in human liver-derived cell line Hep3B

The human liver-derived cell line Hep3B (purchased from ATCC) was suspended in medium A (a medium prepared by dissolving 1% penicillin-streptomycin (Life Technologies Corporation), non-essential amino acids (Life Technologies Corporation), and 10% FBS (Life Technologies Corporation) in MEM (Life Technologies Corporation) and seeded to a 96-well plate (Becton, Dickinson and Company) at a density of 3×10³ cells/well, and cultured at 37°C.
An oligonucleotide was introduced into the Hep3B cells cultured for 24 hours, using DharmaFECT 1 (Thermo Fisher Scientific). Specifically, DharmaFECT 1 was diluted 100 fold with Opti-MEM (Life Technologies Corporation) and allowed to stand for 5 minutes; whereas each oligonucleotide (TUP#003 - TUP#033) was diluted with Opti-MEM. A mixed liquid containing the dilutions of DharmaFECT 1 and the oligonucleotide in a 1:1 ratio was prepared, and allowed to stand for 20 minutes. Transfection was achieved by adding the mixed liquid in an amount 1/10 of that of medium A to the culture broth of Hep3B. The oligonucleotide used for the transfection was prepared so that the concentration thereof would be 30 nM after the addition.
The transfected Hep3B was cultured for 48 hours, after which RNA was extracted from the cells using Cells-to-Ct (Life Technologies Corporation). A cDNA was synthesized using the RNA extracted, and the amount of FGF21 mRNA expressed was quantified by quantitative real time PCR.

The probe and primer sequences used for this study are shown below.
primer for human β-actin:
Forward: 5'-CCTGGCACCCAGCACAAT-3' (SEQ ID NO: 27)
Reverse: 5'-GCCGATCCACACGGAGTACT-3' (SEQ ID NO: 28)
probe for human β-actin:
5'-Fam-ATCAAGATCATTGCTCCTCCTGAGCGC-3' (SEQ ID NO: 29)

primer for human FGF21:
Forward: 5'-CAGCGGTACCTCTACACAGATGA-3' (SEQ ID NO: 30)
Reverse: 5'-CGTCCCATCCTCCCTGATC-3' (SEQ ID NO: 31)
probe for human FGF21:
5'-Fam-CCCAGCAGACAGAAG-3' (SEQ ID NO: 32)

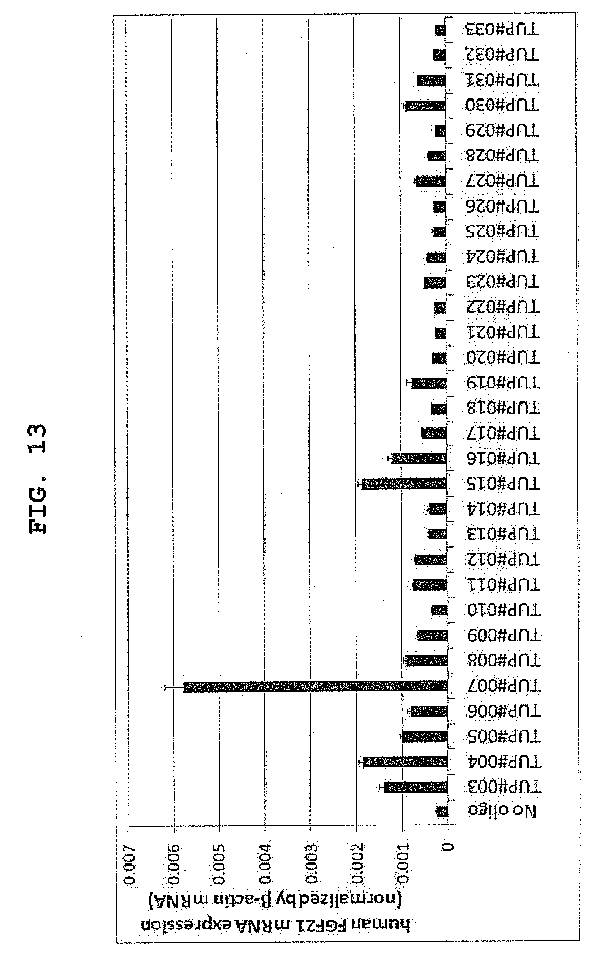

The results of the real time PCR are shown in Figure 13. The amount of FGF21 mRNA is expressed as a value standardized with the amount of β-actin mRNA (mean + standard error). This test demonstrated that each oligonucleotide investigated increased FGF21 mRNA in Hep3B cells.

### (Experimental Example 7) Evaluation of FGF21 mRNA expression elevation activity in mouse liver primary culture cells

The liver of a C57BL/6J mouse (male, CLEA Japan, Inc.) was enzymatically digested with Liver Perfusion Medium (Life Technologies Corporation) and 0.04% collagenase solution (a solution of 0.06 g of collagenase (Sigma) and 0.11 g of CaCl₂. 2H₂O (Wako) dissolved in 150 ml of Hank's solution (Life Technologies Corporation). The cells dispersed by the enzymatic digestion were washed with Hepatocyte Wash Medium (Life Technologies Corporation). The mouse liver primary culture cells obtained were suspended in medium B (a solution containing 1% penicillin-streptomycin (Life Technologies Corporation), 1% L-glutamine (Life Technologies Corporation), 10 nM insulin (Sigma), 10 nM dexamethasone (Wako), and 10% FBS (Life Technologies Corporation) in Williams' Medium E (Life Technologies Corporation), seeded to a Biocoat Collagen I Cellware 24-well plate (Becton, Dickinson and Company) at a density of 6.25×10⁴ cells/well, and cultured for 24 hours.
After the cultivation, an oligonucleotide was introduced into the cells using DharmaFECT 1. Specifically, DharmaFECT 1 was diluted 50 fold with Opti-MEM (Life Technologies Corporation) and allowed to stand for 5 minutes, whereas the oligonucleotide was diluted with Opti-MEM. A mixed liquid containing the dilutions of DharmaFECT 1 and the oligonucleotide in a 1:1 ratio was prepared, and allowed to stand for 20 minutes. Transfection was achieved by adding the mixed liquid in an amount 1/10 of that of medium B to the culture broth of mouse liver primary culture cells. The oligonucleotide used for the transfection was prepared so that the concentration thereof would be 3, 10, or 30 nM after the addition.
The mouse liver primary culture cells transfected were cultured 48 hours, after which RNA was extracted using SV96 total RNA isolation system (Promega). A cDNA was synthesized using the extracted RNA, and the amount of FGF21 mRNA expressed was quantified by quantitative real time PCR.

The probe and primer sequences used for this study are shown below.
primer for mouse β-actin:
Forward: 5'-CACTATTGGCAACGAGCGG-3' (SEQ ID NO: 33)
Reverse: 5'-TCCATACCCAAGAAGGAAGGC-3' (SEQ ID NO: 34)
probe for mouse β-actin:
5'-Fam-TCCGATGCCCTGAGGCTCTTTTCC-3' (SEQ ID NO: 35)

primer for mouse FGF21:
Forward: 5'-GTTTCTTTGCCAACAGCCAGAT-3' (SEQ ID NO: 36)
Reverse: 5'-CCAGCAGCAGTTCTCTGAAGCT-3' (SEQ ID NO: 37)
probe for mouse FGF21:
5'-Fam-ACTTTGATCCTGAGGCCT-3' (SEQ ID NO: 38)

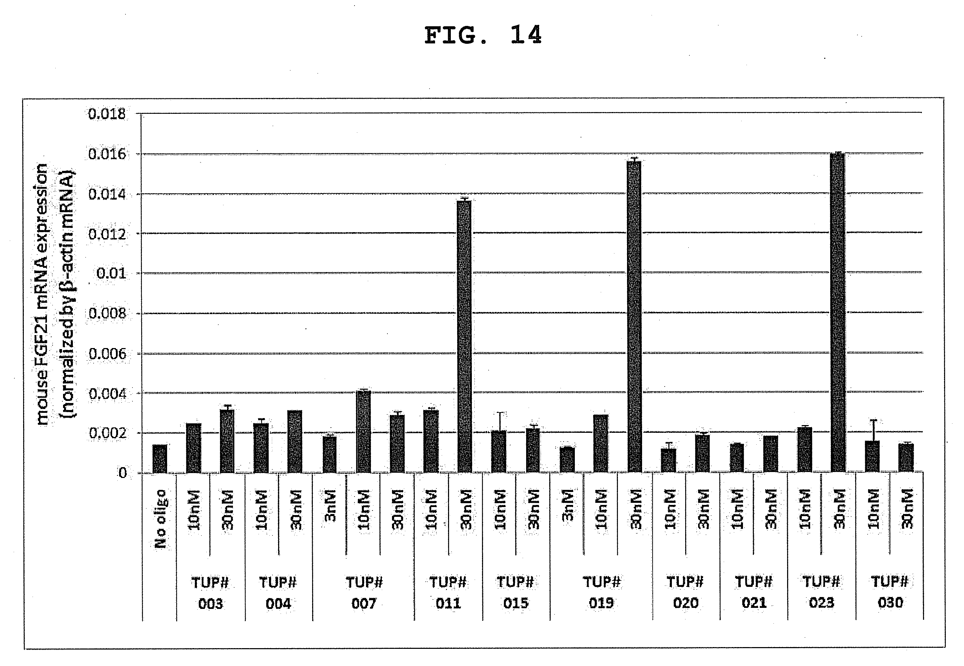

The results of the quantitative real time PCR are shown in Figure 14. The amount of FGF21 mRNA is expressed as a value standardized with the amount of β-actin mRNA (mean + standard error). This test demonstrated that each oligonucleotide investigated increased FGF21 mRNA in mouse liver primary culture cells.

### (Experimental Example 8) Evaluation of FGF21 protein secretion promoting activity in human liver-derived cell line Hep3B

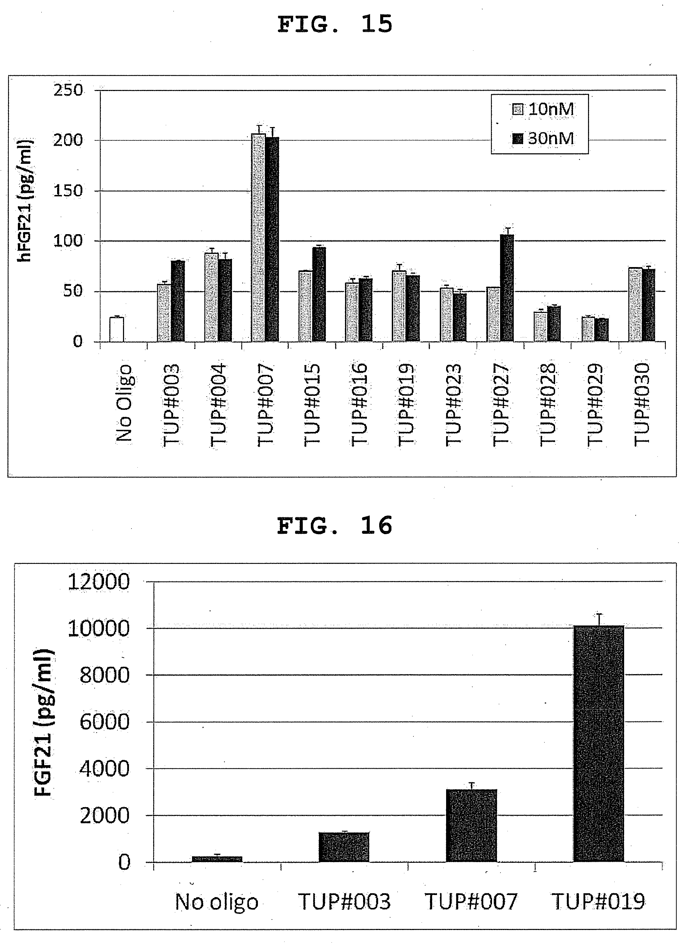

As in Experimental Example 6, Hep3B cells were seeded to a 96-well plate at a density of 3×10³ cells/well. After cultivation for 24 hours, an oligonucleotide was introduced in the same manner as Experimental Example 6 using DharmaFECT 1. The oligonucleotide concentration was adjusted so that it would be 10 or 30 nM after the addition. The Hep3B cells transfected were cultured for 48 hours, after which the culture supernatant was recovered. The amount of FGF21 protein contained in the recovered culture supernatant was measured using a Human FGF21 ELISA Kit (Millipore).
The results are shown in Figure 15 (mean + standard error). This test demonstrated that each oligonucleotide investigated increased the amount of FGF21 protein secreted in Hep3B cells.

### (Experimental Example 9) Evaluation of FGF21 protein secretion promoting activity in mouse liver primary culture cells

Mouse liver primary culture cells were prepared in the same manner as Experimental Example 7; the liver primary culture cells obtained were suspended in Williams' Medium E (Life Technologies Corporation), seeded to a Biocoat Collagen I Cellware 24-well plate (Becton, Dickinson and Company) at a density of 6.25×10⁴ cells/well, and cultured for 24 hours. An oligonucleotide was introduced in the same manner as Experimental Example 7. The oligonucleotide used for the introduction was adjusted so that the concentration thereof would be 30 nM after the addition. The mouse liver primary culture cells transfected were cultured for 48 hours, after which the culture supernatant was recovered.
The amount of FGF21 protein in the culture supernatant was measured using a Rat/Mouse FGF21 ELISA Kit (Millipore).
The results are shown in Figure 16 (mean + standard error). This test demonstrated that each oligonucleotide investigated increased the amount of FGF21 protein secreted in mouse liver primary culture cells.

### (Experimental Example 10) Evaluation of FGF21 mRNA expression elevation activity in mouse liver

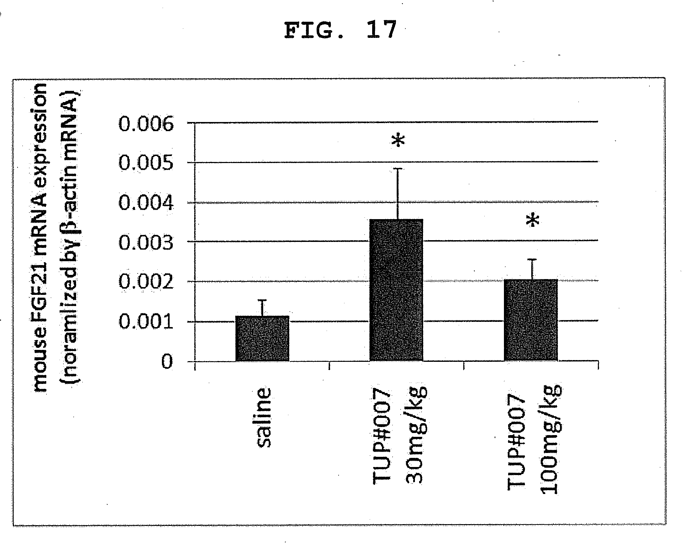

Physiological saline or TUP#007 was intraperitoneally administered to C57BL/6J mice (male, 8-week old, CLEA Japan) at 30 mg/kg or 100 mg/kg; 48 hours later, the livers were collected (n=9 in each group). RNA was extracted from the collected liver using TRIzol (Life Technologies Corporation). A cDNA was synthesized using the RNA obtained, and the amount of FGF21 mRNA expressed was quantified by quantitative real time PCR.
Used in this investigation were the primers and probes described in Experimental Example 7 above.
The results are shown in Figure 17. The amount of FGF21 mRNA is expressed as a value standardized with the amount of β-actin mRNA (mean + standard error). In the drawing, * indicates the results of Shirley-Williams test, showing that the amount of FGF21 mRNA expressed increased significantly (p<0.05) in the TUP#007 dosed group compared with the no-oligonucleotide dosed group.
This result demonstrated that by acquiring a substance that binds to a cis-element of FGF21 mRNA to inhibit the binding of a cis-element binding factor to the cis-element, the amount of FGF21 mRNA expressed and the amount of FGF21 protein secreted can be controlled at the culture cell level and the individual level.

### Industrial Applicability

Since the substance of the present invention, which is capable of binding to a cis-element of the mRNA of FGF21 to inhibit the binding of a cis-element binding factor to the cis-element, can enhance the translation level of FGF21 protein by stabilizing said mRNA, it is useful as a drug for the prophylaxis or treatment of various lifestyle-related diseases for which FGF21 shows a prophylactic or therapeutic effect.

The contents disclosed in any publication cited herein, including patents and patent application specifications, are hereby incorporated in their entireties by reference, to the extent that they have been disclosed herein.

The present invention is based on a patent application No. 2009-172584 filed in Japan (filing date: July 23, 2009), the contents of which are incorporated in full herein.

## Claims

1. A substance capable of binding to at least a portion of a cis-element of the mRNA of FGF21 to inhibit the binding of a cis-element binding factor to the cis-element.

2. The substance according to claim 1, which is capable of binding to at least a portion of a cis-element of the mRNA of FGF21 to inhibit the binding of an AU-rich element binding factor to an AU-rich element.

3. The substance according to claim 1, wherein the cis-element of the mRNA of FGF21 is the base sequence of the 835th to 842nd bases from the 5'-end side of the base sequence of the mRNA of human FGF21.

4. The substance according to claim 1, wherein the substance is a nucleic acid analogue, or a salt thereof, having a base sequence complementary to at least a portion of a cis-element of the mRNA of FGF21 and a base sequence complementary to a portion of the untranslated region on the 5' side and/or 3' side of the cis-element, and capable of inhibiting the binding of a cis-element binding factor to the cis-element by binding specifically to at least a portion of the cis-element on the basis of the sequence specificity possessed by the base sequence complementary to the untranslated region.

5. The substance according to claim 1, wherein the substance is oligonucleotide, or a salt thereof, having a base sequence complementary to at least a portion of a cis-element of the mRNA of FGF21 and a base sequence complementary to a portion of the untranslated region on the 5' side and/or 3' side of the cis-element, and capable of inhibiting the binding of a cis-element binding factor to the cis-element by binding specifically to at least a portion of the cis-element on the basis of the sequence specificity possessed by the base sequence complementary to the untranslated region.

6. The substance according to claim 5, wherein the substance comprises an LNA (locked nucleic acid).

7. The substance according to claim 5, comprising a BNA (bridged nucleic acid).

8. The substance according to claim 1, wherein the substance is an oligonucleotide containing a base sequence shown by SEQ ID NO:3, SEQ ID NO:18, SEQ ID NO:19, SEQ ID NO:20, SEQ ID NO:21, SEQ ID NO:22, SEQ ID NO:23 or SEQ ID NO:24 or substantially the same base sequence as the base sequence or a salt thereof.

9. The substance described in any one of claims 4 to 7, wherein the substance is an oligonucleotide containing a base sequence shown by SEQ ID NO:3 or substantially the same base sequence as the base sequence, or a salt thereof.

10. The substance described in any one of claims 4 to 7, which is an oligonucleotide containing a base sequence shown by SEQ ID NO:19 or a base sequence substantially the same as the base sequence, or a salt thereof.

11. The substance according to claim 1, which is an oligonucleotide represented by TUP#001, TUP#002, TUP#003, TUP#004, TUP#005, TUP#006, TUP#007, TUP#008, TUP#009, TUP#010, TUP#011, TUP#012, TUP#013, TUP#014, TUP#015, TUP#016, TUP#017, TUP#018, TUP#019, TUP#020, TUP#021, TUP#022, TUP#023, TUP#024, TUP#025, TUP#026, TUP#027, TUP#028, TUP#029, TUP#030, TUP#031, TUP#032 or TUP#033, or a salt thereof.

12. A medicament comprising the substance according to claim 1.

13. The medicament according to claim 12, which is an agent for the prophylaxis or treatment of a lifestyle-related disease.

14. A method for the prophylaxis or treatment of a lifestyle-related disease in a mammal, comprising administering an effective amount of the substance according to claim 1 to the mammal.

15. Use of the substance according to claim 1 for the production of an agent for the prophylaxis or treatment of a lifestyle-related disease.

16. A carrier comprising the substance according to claims 5, 8, 9, 10 or 11.

17. A medicament comprising a combination of two or more of substances capable of binding to at least a portion of a cis-element of the mRNA that encodes each of two different kinds or more of target proteins to inhibit the binding of a cis-element binding factor to the cis-element.

18. The medicament according to claim 17, wherein the substance is a substance capable of binding to at least a portion of a cis-element of the mRNA that encodes each target protein to inhibit the binding of an AU-rich element binding factor to the AU-rich element.

19. The medicament according to claim 17, wherein the substance is a nucleic acid analogue, or a salt thereof, having a base sequence complementary to at least a portion of a cis-element of the mRNA that encodes each target protein and a base sequence complementary to a portion of the untranslated region on the 5' side and/or 3' side of the cis-element, and capable of inhibiting the binding of a cis-element binding factor to the cis-element by binding specifically to at least a portion of the cis-element on the basis of the sequence specificity possessed by the base sequence complementary to the untranslated region.

20. The medicament according to claim 17, wherein the substance is an oligonucleotide, or a salt thereof, having a base sequence complementary to at least a portion of a cis-element of the mRNA that encodes each target protein and a base sequence complementary to a portion of the untranslated region on the 5' side and/or 3' side of the cis-element, and capable of inhibiting the binding of a cis-element binding factor to the cis-element by binding specifically to at least a portion of the cis-element on the basis of the sequence specificity possessed by the base sequence complementary to the untranslated region.

21. The medicament according to claim 20, wherein the substance is an LNA-containing oligonucleotide or a salt thereof.

22. The medicament according to claim 20, wherein the substance is a BNA-containing oligonucleotide or a salt thereof.

23. An agent for the prophylaxis or treatment of a lifestyle-related disease, comprising a substance inhibiting the function of ZFP36L1 or ZFP36L2.

24. A method for the prophylaxis or treatment of a lifestyle-related disease in a mammal, comprising administering an effective amount of the substance according to claim 23 to the mammal.

25. Use of the substance according to claim 23 for the production of an agent for the prophylaxis or treatment of a lifestyle-related disease.
